# EUROPEAN PATENT SPECIFICATION

(11) **EP 1 372 682 B1**
(45) Date of publication and mention of the grant of the patent: **02.05.2012**
(21) Application number: 02721413.9
(22) Date of filing: 15.03.2002
(51) Int. Cl.: A61K 39/385, A61K 31/353

(54) **CATECHINS FOR THE TREATMENT OF FIBRILLOGENESIS IN ALZHEIMER'S DISEASE, PARKINSON'S DISEASE, SYSTEMIC AA AMYLOIDOSIS, AND OTHER AMYLOID DISORDERS**
CATECHINE ZUR BEHANDLUNG DER FIBRILLOGENESE BEI ALZHEIMER-KRANKHEIT, PARKINSON-KRANKHEIT, SYSTEMISCHER AA-AMYLOIDOSIS UND ANDERER AMYLOID-STÖRUNGEN
CATECHINES DESTINEES AU TRAITEMENT DE LA FIBRILLOGENESE DANS LA MALADIE D'ALZHEIMER, LA MALADIE DE PARKINSON, L'AMYLOSE AA SYSTEMIQUE, ET AUTRES TROUBLES AMYLOIDES

(30) Priority: 15.03.2001 US 276866 P; 10.12.2001 US 338969 P
(43) Date of publication of application: 02.01.2004
(73) Proprietor: Proteotech Inc., Kirkland, WA 98034 (US)
(72) Inventor: CASTILLO, Gerardo, M., Seattle, WA 98178 (US); CHOI, Paula, Y., Bellevue, WA 98007 (US); CUMMINGS, Joel, A., Seattle, WA 98115 (US); NGUYEN, Beth, P., Bothell, WA 98012-6236 (US); SNOW, Alan, D., Lynnwood, WA 98037 (US)
(74) Representative: Lee, Nicholas John
(86) International application number: PCT/US2002/007755
(87) International publication number: WO 2003/013442

(56) References cited:
- WO-A-01/49281
- WO-A-01/49307
- WO-A-96/28178
- WO-A1-98/51302
- PATENT ABSTRACTS OF JAPAN vol. 1998, no. 14, 31 December 1998 (1998-12-31) & JP 10 245342 A (MITSUI NORIN KK), 14 September 1998 (1998-09-14)
- YOUNG-TAEG C ET AL: "The green tea polyphenol (-)-epigallocatechin gallate attenuates beta-amyloid-induced neurotoxicity in cultured hippocampal neurons" LIFE SCIENCES, vol. 70, no. 5, 21 December 2001 (2001-12-21), pages 603-614, XP002341713

## Description

### TECHNICAL FIELD

The invention relates to compositions for treating Lewy body disease or Parkinson's disease, using catechins.

### BACKGROUND OF THE INVENTION

Alzheimer's disease is characterized by the accumulation of a 39-43 amino acid peptide termed the beta-amyloid protein or Aß, in a fibrillar form, existing as extracellular amyloid plaques and as amyloid within the walls of cerebral blood vessels. Fibrillar Aß amyloid deposition in Alzheimer's disease is believed to be detrimental to the patient and eventually leads to toxicity and neuronal cell death, characteristic hallmarks of Alzheimer's disease. Accumulating evidence implicates amyloid as a major causative factor of Alzheimer's disease pathogenesis.

Parkinson's disease is another human disorder characterized by the formation, deposition, accumulation and/or persistence of abnormal fibrillar protein deposits that demonstrate many of the characteristics of amyloid. In Parkinson's disease, an accumulation of cytoplasmic Lewy bodies consisting of filaments of alpha-synuclein/ NAC are believed important in the pathogenesis and as therapeutic targets. New agents or compounds able to inhibit alpha-synuclein/NAC formation, deposition, accumulation and/or persistence, or disrupt pre-formed alpha-synuclein/NAC fibrils (or portions thereof) are regarded as potential therapeutics for the treatment of Parkinsons disease.

A variety of other human diseases also demonstrate amyloid deposition and usually involve systemic organs (i.e. organs or tissues lying outside the central nervous system), with the amyloid accumulation leading to organ dysfunction or failure. These amyloid diseases (discussed below) leading to marked amyloid accumulation in a number of different organs and tissues are known as systemic amyloidoses. In other amyloid diseases, single organs may be affected such as the pancreas in 90% of patients with type 2 diabetes. In this type of amyloidosis, the beta-cells in the islets ofLangerhans in pancreas are believed to be destroyed by the accumulation of fibrillar amyloid deposits consisting primarily of a protein known as islet amyloid polypeptide (IAPP). Inhibiting or reducing such amyloid accumulation is believed to lead to new effective treatments for type 2 diabetes. In Alzheimer's disease, Parkinson's and "systemic" amyloid diseases, there is currently no cure or effective treatment, and the patient usually dies within 3 to 10 years from disease onset.

The amyloid diseases include, but are not limited to, the amyloid associated with Alzheimer's disease, Down's syndrome, hereditary cerebral hemorrhage with amyloidosis of the Dutch type, and inclusion body myositosis (Askanas et al, Ann. Neurol. 43:521-560, 1993) (wherein the specific amyloid is referred to as beta-amyloid protein or AB), the amyloid associated with chronic inflammation, various forms of malignancy and Familial Mediterranean Fever (wherein the specific amyloid is referred to as AA amyloid or inflammation-associated amyloidosis), the amyloid associated with multiple myeloma and other B-cell dyscrasias (wherein the specific amyloid is referred to as AL amyloid), the amyloid associated with type II diabetes (wherein the specific amyloid protein is referred to as amylin or islet amyloid polypeptide), the amyloid associated with the prion diseases including Creutzfeldt-Jakob disease, Gerstmann-Straussler syndrome, kuru and animal scrapie (wherein the specific amyloid is referred to as PrP amyloid), the amyloid associated with long-term homodialysis and carpal tunnel syndrome (wherein the specific amyloid is referred to as beta₂-microglobulin amyloid), the amyloid associated with senile cardiac amyloid and Familial Amyloidotic Polyneuropathy (wherein the specific amyloid is referred to as transthyretin or prealbumin), and the amyloid associated with endocrine tumors such as medullary carcinoma of the thyroid (wherein the specific amyloid is referred to as variants of procalcitonin).

In addition, the alpha-synuclein protein which forms fibrils, and is Congo red and Thioflavin S positive, is found as part of Lewy bodies in the brains of patients with Parkinson's disease, Lewy body disease (Lewy in Handbuch der Neurologie, M. Lewandowski, ed., Springer, Berline pp.920-983, 1912; Pollanen et al, J. Neuropath, Exp. Neurol. 52:183-191, 1993; Spillantini et al, Proc, Natl. Acad. Sci. USA 96:6469-6473, 1998; Arai et al, Neurosc. Lett. 269:83-86, 1999), and multiple system atrophy (Wakabayashi et al, Acta Neuropath. 96:445-452, 1998).

Discovery and identification of new compounds or agents as potential therapeutics to arrest amyloid formation, deposition, accumulation and/or persistence that occurs in Alzheimer's disease, Parkinson's disease, systemic AA amyloidosis, type 2 diabetes, and other amyloidoses are desperately sought.

### DISCLOSURE OF THE INVENTION

In a first aspect, the present invention provides the use of a catechin in the manufacture of a medicament for treating Parkinson's disease or Lewy body disease, wherein the catechin is selected from the group consisting of catechin, epicatechin, gallocatechin gallate, epigallocatechin gallate, epigallocatechin, and epicatechin gallate, and pharmaceutically acceptable salts of the foregoing catechins.

In a second aspect, the present invention provides a catechin selected from the group consisting of catechin, epicatechin, gallocatechin gallate, epigallocatechin gallate, epigallocatechin, and epicatechin gallate, and pharmaceutically acceptable salts of the foregoing catechins, for treating Parkinson's disease or Lewy body disease.

The medicament may be for oral administration, parenteral injection, intravenous injection, subcutaneous injection, intramuscular injection, topical administration, or aerosol spray administration.

In a third aspect, the present invention provides a method of treatment of alpha-synuclein or NAC fibrillogenesis in an *in vitro* environment, the method comprising the step of administering into the *in vitro* environment a therapeutically effective amount of a catechin selected from the group consisting of catechin, epicatechin, gallocatechin gallate, epigallocatechin gallate, epigallocatechin, and epicatechin gallate, and pharmaceutically acceptable salts of the foregoing catechins.

In a fourth aspect, the present invention provides a pharmaceutical composition for use in treating Parkinson's disease or Lewy body disease, comprising a therapeutically effective amount of a catechin selected from the group consisting of catechin, epicatechin, gallocatechin gallate, epigallocatechin gallate, epigallocatechin, and epicatechin gallate, and pharmaceutically acceptable salts of the foregoing catechins and a pharmaceutically acceptable carrier, diluent, or excipient, wherein the therapeutically effective amount of the catechin comprises a dosage in the range of about 0.1 to 500 mg/kg of body weight of the subject.

The therapeutically effective amount of the catechin may comprise a dosage in the range of about 1.0 to 100 mg/kg of body weight of the subject.

In certain embodiments, the composition comprises a mixture of two or more of the catechins selected from the group consisting of catechin, epicatechin, gallocatechin gallate, epigallocatechin gallate, epigallocatechin, and epicatechin gallate, and pharmaceutically acceptable salts of the foregoing catechins.

The catechin selected is preferably in substantially pure isolated form.

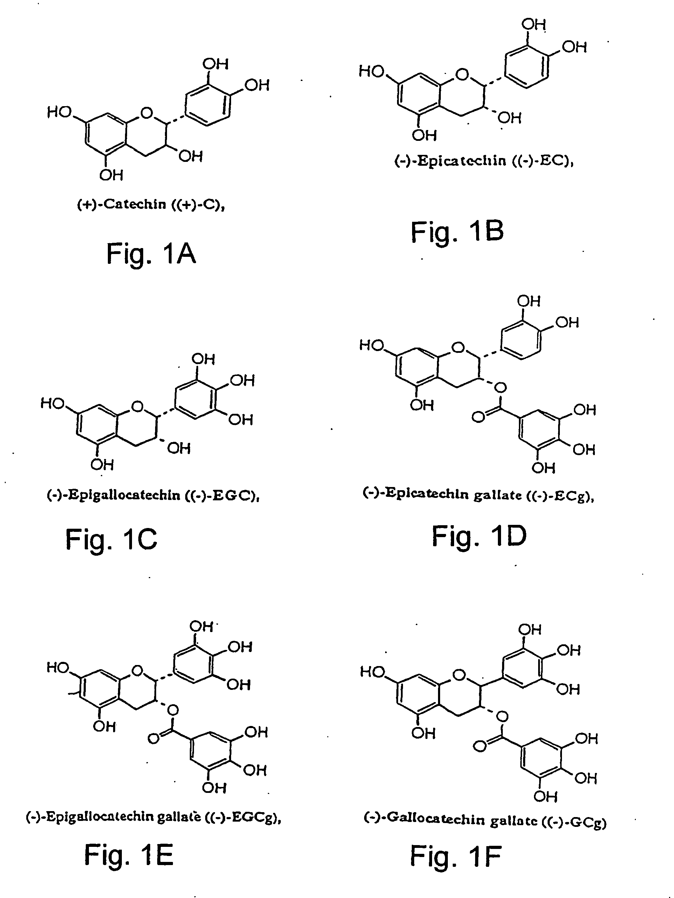

The identification and use of standardized green tea extract and derivatives and constituents thereof, such as the catechin compounds shown for example in Figure 1, are disclosed for the therapeutic intervention of Alzheimer's disease, systemic AA amyloidosis and other amyloidoses, and Parkinson's and Lewy body diseases.

In addition, methods of isolation for the identification and purification of the potent amyloid inhibitory ingredients within green tea extract are disclosed. Use of standardized green tealeaf extract and its ingredients (*i.e.* 60% polyphenols) contained within different commercial preparations are anticipated to benefit human patients with Alzheimer's disease and other amyloidoses, and Parkinson's and Lewy body diseases, due to green tea leaf extract's ability to inhibit amyloid fibril formation, and Parkinson's alpha-synuclein fibril and Lewy body formation, and to cause dissolution/ disruption, and disaggregation of pre-formed amyloid and alpha-synuclein fibrils.

We disclose the identification of and make the surprising discovery that commercially available catechins, such as those present in standardized green tea extracts, and the standardized green tea leaf extract itself (such as found in extract standardized to 50% polyphenols) act as an impressive inhibitor of Alzheimer's disease amyloid fibril formation, systemic AA amyloid fibril formation, and Parkinson's disease alpha-synuclein/NAC fibril formation. Furthermore, commercially available catechins (including but not limited to epicatechin, catechin, gallocatechin gallate, epigallocatechin gallate, epigallocatechin, and/or epicatechin gallate) and standardized green tea leaf extract have the ability to cause a disassembly/disruption of pre-formed amyloid fibrils of the Alzheimer's type, suggesting that this agent may be useful for patients at latter stages Alzheimer's disease, and those affected with other amyloid diseases. Standardized green tea leaf extract and catechins obtained from different commercial sources (extracts isolated from gelatin-coated capsules) were found to serve as potent inhibitors of Alzheimer's disease amyloid fibrillogenesis and Parkinson's disease protein fibrillogenesis.

For purposes of this disclosure, Parkinson's disease, due to the fact that fibrils develop in the brains of patients with this disease (which are Congo red and Thioflavin S positive, and which contain predominant beta-pleated sheet secondary structure), is identified and discussed as a disease which also displays the characteristics of an amyloid-like disease, and disclosures herein related to amyloidoses are therefore expected in like manner to relate therapeutically to Parkinson's and Lewy body diseases. Therefore agents or compounds found to inhibit Alzheimer's disease Aβ amyloid fibril formation, are anticipated to also be effective in the inhibition of alpha-synuclein/NAC fibril formation. These agents or compounds will therefore also serve as therapeutics for Parkinson's and Lewy body diseases, in addition to having efficacy as a therapeutic for Alzheimer's disease, systemic amyloidosis, type 2 diabetes, and other amyloid disorders.

Selected polyhydroxylated aromatic compounds, such as epicatechin, appear to be effective as inhibitors of systemic AA amyloidosis *in vivo.* Significantly, we have discovered that catechin itself (the epimer of epicatechin) is not effective as an inhibitor of systemic AA amyloidosis. We identify various polyhydroxylated aromatic compounds that inhibit fibrillar AA amyloid formation and/or deposition and that are useful for the treatment of systemic AA amyloidosis found in patients with chronic inflammatory disorders.

Both commercially available standardized green tea leaf extract and various catechins caused a marked significant dose-dependent inhibition of Aß 1-40 amyloid fibril formation as determined using a Thioflavin T fluorometry assay in a dose-dependent manner. Standardized green tea leaf extract and catechins obtained from commercial sources were also potent disrupters of pre-formed Aß 1-42 containing amyloid fibrils, as determined using a Thioflavin T fluorometry assay, and exerted their effects in a dose-dependent manner. Selected polyhydroxylated aromatic compounds, such as epicatechin appear to be effective inhibitors of systemic AA amyloidosis in an experimental mouse model. Lastly, standardized green tea leaf extract obtained from different commercial sources caused a disaggregation of pre-formed Aß 1-42 Alzheimer's amyloid fibrils, and catechins obtained from different commercial sources caused an inhibition of NAC-containing Parkinson's disease fibrils.

Therefore, the use of standardized green tea leaf extract and/or specific catechins (in various forms, i.e. a pill, tablet, liquid form, powder form, etc.) and derivatives thereof, from different commercial sources is disclosed for the treatment of fibrillogenesis in Alzheimer's disease, Parkinson's disease, systemic AA amyloidosis, and other amyloidoses. Also disclosed are methods of isolation to identify and purify the key amyloid inhibitory ingredients within the green tea extract material. Identification of the "active" amyloid inhibitory ingredients within the green tea extracted plant materials are anticipated to lead to new drug design for anti-amyloid therapeutics of the future. Current use of standardized green tea leaf extract and its ingredients, such as catechins as contained within different commercial preparations, are anticipated to benefit human patients at all stages of Alzheimer's, Parkinson's and other amyloid or amyloid-like diseases due to catechin's and standardized green tea extract's newly demonstrated ability to inhibit Aß amyloid fibril formation, and alpha-synuclein fibril formation (important for treating early to mid-stage Alzheimer's disease and Parkinson's disease, respectively), and cause dissolution/disruption and disaggregation of pre-formed amyloid fibrils (important for treating mid to late stages of Alzheimer's disease and Parkinson's disease, respectively). Similarly, catechins and standardized green tea leaf extract are anticipated to benefit patients with different systemic amyloid diseases such as systemic AA amyloidosis, and type 2 diabetes, regardless of the stage of amyloid accumulation and the organ (or tissue) involved.

While tea extract results are exemplified with Species Camellia sinensis, extracts from other species within the family Theaceae are believed to have similar effects.

The use of green tea, green tea leaves and extracts, and catechins. or derivatives thereof, is disclosed for the treatment of amyloid and/or fibril formation, deposition, accumulation and/or persistence in Alzheimer's disease, Parkinson's disease, systemic AA amyloidosis, type 2 diabetes and other amyloidoses.

"Treatment" is also intended in every possible instance to include and cover "*in vitro* treatment", whether for experimental or screening purposes and the like, and whether or not the *in vitro* treatment leads to, or is ever intended to lead to, treatment of a like fibillogenesis, or any amyloid or alpha-synuclein disease corresponding to that fibrillogenesis, in a mammalian subject.

Also disclosed is the use of green tea, green tea leaves and extracts or derivatives thereof, from the Camellia sinensis species for the treatment of amyloid formation, deposition, accumulation and/or persistence in Alzheimer's disease, Parkinson's disease, systemic AA amyloidosis, type 2 diabetes and other amyloidoses.

Green tea, green tea leaves and extracts or derivatives thereof, from the Theaceae family are disclosed for the treatment of amyloid formation, deposition, accumulation and/or persistence in Alzheimer's disease, Parkinson's disease, systemic AA amyloidosis, type 2 diabetes and other amyloidoses.

Commercially available pills, tablets, caplets, soft and hard gelatin capsules, lozenges, sachets, cachets, vegicaps, liquid drops, elixers, suspensions, emulsions, solutions, syrups, tea bags, tea leaves, aerosols (as a solid or in a liquid medium), suppositories, sterile injectable solutions, sterile packaged powders, and/or leaf powder which contain green tea, green tea leaves and extracts or derivatives thereof, are disclosed for the treatment of patients with Alzheimer's disease, Parkinson's disease, systemic AA amyloidosis, type 2 diabetes and other amyloidoses.

Green tea, green tea leaves and extracts or derivatives thereof, and/or the polyphenols contained within green tea, green tea leaves and extracts or derivatives thereof, for the treatment of amyloid formation, deposition, accumulation and/or persistence in Alzheimer's disease, Parkinson's disease, systemic AA amyloidosis, type 2 diabetes and other amyloidoses.

The invention is described with reference to specific embodiments, plant species and parts, methods, procedures and the like. However, it will be recognized by those skilled in the art that various chemical substitutions can be made within the disclosed compounds without departing from the spirit and scope of the invention. In particular, it is known that catechins can be isolated and/or purified from plant materials by a number of different methods. It will further be recognized that these alternate methods, and consequent changes in other steps of the method, such as use of different solvents or different columns for purification, and of catechins from a composition of partially purified polyphenols, fall within the scope of the presently disclosed plant-derived extracts, and compounds derived thereof.

Also disclosed is the use of catechins contained within green tea, green tea leaves and extracts or derivatives thereof, for the treatment of amyloid formation, deposition, accumulation and/or persistence in Alzheimer's disease, Parkinson's disease, systemic AA amyloidosis, type 2 diabetes and other amyloidoses.

Catechins, including but not limited to, catechin, epicatechin, gallocatechin gallate, epigallocatechin gallate, epigallocatechin, and/or epicatechin gallate, whether contained within green tea, green tea leaves and extracts or derivatives thereof, or from other natural or synthetic sources, are disclosed for the treatment of amyloid formation, deposition, accumulation and/or persistence in Alzheimer's disease, Parkinson's disease, systemic AA amyloidosis, type 2 diabetes and other amyloidoses.

Also disclosed is the use of bioflavanoids contained within green tea, green tea leaves and extracts or derivatives thereof, for the treatment of amyloid formation, deposition, accumulation and/or persistence in Alzheimer's disease, Parkinson's disease, systemic AA amyloidosis, type 2 diabetes and other amyloidoses, and the use of flavanols contained within green tea, green tea leaves and extracts or derivatives thereof, for the treatment of amyloid formation, deposition, accumulation and/or persistence in Alzheimer's disease, Parkinsons disease, systemic AA amyloidosis, type 2 diabetes and other amyloidoses.

Also disclosed is the use of flavandiols contained within green tea, green tea leaves and extracts or derivatives thereof, for the treatment of amyloid formation, deposition, accumulation and/or persistence in Alzheimer's disease, Parkinson's disease, systemic AA amyloidosis, type 2 diabetes and other amyloidoses, and the use of flavanoids contained within green tea, green tea leaves and extracts or derivatives thereof, for the treatment of amyloid formation, deposition, accumulation and/or persistence, in Alzheimer's disease, Parkinson's disease, systemic AA amyloidosis, type 2 diabetes and other amyloidoses.

Also disclosed is the use of tannins contained within green tea, green tea leaves and extracts or derivatives thereof, for the treatment of amyloid formation, deposition, accumulation and/or persistence in Alzheimer's disease, Parkinson's disease, systemic AA amyloidosis, type 2 diabetes and other amyloidoses.

Methods to isolate the active ingredients present within green tea, green tea leaves and extracts or derivatives thereof, are also disclosed for use as potent agents which inhibit amyloid formation, amyloid deposition, amyloid accumulation, amyloid persistence, cause a disassembly/disruption and/or cause a disassembly of pre-formed or pre-deposited amyloid fibrils in Alzheimer's disease, Parkinson's disease, systemic AA amyloidosis, type 2 diabetes and other amyloidoses. Methods for isolation of the active ingredients within green tea, green tea leaves and extracts or derivatives thereof, include application of some standard techniques known to those skilled in the art, including, but not limited to, thin layer chromatography using silica-coated plates, and separation and isolation using high or low pressure liquid chromatography (HPLC). Other active ingredients within green tea, green tea leaves and extracts or derivatives thereof, found to be potent inhibitors of amyloid formation, amyloid deposition, amyloid accumulation, amyloid persistence, and/or cause a disassembly/disruption, and disaggregation of pre-formed or pre-deposited amyloid fibrils in Alzheimer's disease, Parkinson's disease, systemic AA amyloidosis, type 2 diabetes and other amyloidoses, are identified by re-testing of individual bands or fractions (separated by thin layer chromatography, column chromatography and/or HPLC) using specific assay tests as described in the Examples.

Sufficient isolation of these active ingredients contained within individual bands and/or fractions are then sent out for specific analyses which may include, but are not limited to, scanning electron microscope equipped with energy dispersive x-ray analyzer to detect and spatially map some elements present in each sample, elemental analysis by combustion to determine the relative % of carbon, hydrogen and nitrogen, high resolution mass spectroscopy to determine molecular weight and elemental composition, Fourier transform infrared spectroscopy to determine functional groups and make comparisons to the spectra of known compounds, differential scanning calorimetry to determine melting point, atomic absorption, gel chromatography, high performance liquid chromatography, proton and C¹⁸ nuclear magnetic resonance spectroscopy for material characterization and to provide information regarding the position of atoms relative to each other, and UV/VIS spectroscopy. It is expected that additional techniques will be developed as part of the further isolation of potent active ingredients within green tea, green tea leaves and extracts or derivatives thereof.

The use of green tea, green tea leaves and extracts or derivatives thereof, and/or its ingredients [(regardless of commercial source and regardless of final form for consumption by humans, i.e. pills, tablets, caplets, soft and hard gelatin capsules, lozenges, sachets, cachets, vegicaps, liquid drops, elixers, suspensions, emulsions, solutions, syrups, tea bags, aerosols (as a solid or in a liquid medium), suppositories, sterile injectable solutions, sterile packaged powders, and/or tea leaf powder] are disclosed for inhibition of amyloid formation, deposition, accumulation, and/or persistence, regardless of its clinical setting.

Compositions and methods involving administering to a subject a therapeutic dose of green tea, green tea leaves and extracts or derivatives thereof, which inhibits amyloid deposition is also disclosed. Accordingly, disclosed compositions and methods are useful for inhibiting amyloidosis in disorders in which amyloid deposition occurs. The compounds disclosed herein can be used therapeutically to treat amyloidosis or can be used prophylactically in a subject susceptible to amyloidosis. Disclosed methods are based, at least in part, in directly inhibiting amyloid fibril formation, causing disassembly/disruption and/or disaggregation of pre-formed amyloid fibrils.

Pharmaceutical compositions for treating amyloidosis are also disclosed. The disclosed pharmaceutical compositions include a therapeutic compound in an amount effective to inhibit amyloid deposition and a pharmaceutically acceptable vehicle.

The use of any and all synthetic compounds that are similar to green tea, green tea leaves, extracts or derivatives thereof and/or its active ingredients, for use as potent agents which inhibit amyloid formation, amyloid deposition, amyloid accumulation, amyloid persistence, cause a disassembly/ disruption, and/or disaggregation of pre-formed or pre-deposited amyloid fibrils in Alzheimer's disease, Parkinson's disease, systemic AA amyloidosis, type 2 diabetes and other amyloidoses, are also disclosed

A method of isolation to purify and identify the amyloid inhibitory ingredients from green tea, green tea leaves and extracts or derivatives thereof is disclosed. In one such method, an extract prepared from commercially obtained pills, tablets, caplets, soft and hard gelatin capsules, lozenges, sachets, cachets, vegicaps, liquid drops, elixers, suspensions, emulsions, solutions, syrups, tea bags, aerosols (as a solid or in a liquid medium), suppositories, sterile injectable solutions, sterile packaged powders, tea powder, using the method employing some or all of the following steps: a) extraction from green tea, green tea leaves and extracts or derivatives thereof regardless of form as described above using water or alcohol (i.e. methanol, ethanol or propanol, b) centrifugation at 2,500X g for 20 minutes and collection of supernatant, c) rotary evaporation to dryness for alcohol-extracted compounds and lyophilization for water-extracted compounds, d) washing dry powder obtained with 4 volumes of petroleum ether (repeated 4 times), followed by centrifugation (each time) at 2,500X g for 20 minutes, and collection of supernatants and pellets, e) air-drying of collected pellets, f) re-extraction of air-dried pellets with water and centrifugation at 2,500X g for 20 minutes, g) lyophilization of collected supernatants (referred to as water extracts), h) re-dissolving the pellets or lyophilized water-extract powder in acetonitrile/water/trifluoroacetic acid (TFA), i) injecting and separation by HPLC or low pressure chromatography, j) identifying amyloid inhibitory ingredients by testing in relevant *in vitro* and *in vivo* assays, and k) sending out for structural analysis and elemental composition, as described herein.

A composition, in the form of a dietary supplement, for providing, supporting or improving in a subject one or more of the mental or cognitive qualities selected from the group of mental or cognitive qualities consisting of nutritional support for age related cognitive or memory decline, normal brain function, cognitive ability, and concentration, wherein the composition comprises green tea, green tea leaves and extracts or derivatives thereof, is also disclosed.

A composition, in the form of a dietary supplement, for promoting, maintaining or enhancing in a subject one or more of the mental or cognitive qualities selected from the group of mental or cognitive qualities consisting of mental acuity, mental alertness, cognitive well being, normal brain function, cognitive ability, mental performance, memory concentration, mental sharpness, mental vitality, mental clarity, short term memory, normal brain function, learning, and good brain health, wherein the composition comprises green tea, green tea leaves and extracts or derivatives thereof, is disclosed.

A composition, in the form of a dietary supplement, for promoting or supporting healthy pancreatic function in a subject, by helping to promote normal insulin function, or for reducing, disrupting, dissolving, inhibiting, eliminating or preventing in a subject one or more conditions involving the pancreas selected from the group of conditions involving the pancreas consisting of amyloid fibril deposits, amyloid protein deposits, pancreas associated with amyloid fibril deposits, pancreas associated amyloid protein deposits, amyloid fibril formation and growth, and pancreas associated amyloid fibril formation and growth, wherein the composition comprises green tea, green tea leaves and extracts or derivatives thereof, is also disclosed.

Use of
(a) epicatechin or derivatives thereof,
(b) catechins, catechin hydrate or derivatives thereof,
(c) gallocatechin gallate or derivatives thereof,
(d) epicatechin gallate or derivatives thereof,
(e) epigallocatechin or derivatives thereof,
(f) epigallocatechin gallate or derivatives thereof,
(g) green tea, green tea leaves and extracts or derivatives thereof,
(h) green tea, green tea leaves and extracts or derivatives thereof, from the Camellia sinensis species,
(i) green tea, green tea leaves and extracts or derivatives thereof, from the Theaceae family,
(j) commercially available pills, tablets, caplets, soft and hard gelatin capsules, lozenges, sachets, cachets, vegicaps, liquid drops, elixers, suspensions, emulsions, solutions, syrups, tea bags, tea leaves, aerosols (as a solid or in a liquid medium), suppositories, sterile injectable solutions, sterile packaged powders, and/or leaf powder which contain green tea, green tea leaves and extracts or derivatives thereof, or catechins, and
(k) catechins, including but not limited to catechin, epicatechin, epigallocatechin, epicatechin gallate, epigallocatechin gallate and gallocatechin gallate contained within green tea, green tea leaves and extracts or derivatives thereof, for the treatment of fibril formation, deposition, accumulation and/or persistence in Alzheimer's disease, Parkinson's disease, systemic AA amyloidosis, type 2 diabetes and other amyloidoses is disclosed.

Compositions and methods involving administering to a subject a therapeutic dose of catechins or derivatives thereof, which inhibits amyloid deposition are also disclosed, Accordingly, disclosed compositions and methods are useful for inhibiting amyloidosis in disorders in which amyloid deposition occurs. The compounds can be used therapeutically to treat amyloidosis or can be used prophylactically in a subject susceptible to amyloidosis, The methods are based, at least in part, in directly inhibiting amyloid fibril formation, causing disassembly/disruption and/or disaggregation of pre-formed amyloid fibrils.

Use of any and all synthetic catechins or derivatives thereof and/or their active ingredients, for use as potent agents which inhibit amyloid formation, amyloid deposition, amyloid accumulation, amyloid persistence, cause a disassembly/ disruption, and/or disaggregation of pre-formed or pre-deposited fibrils in Alzheimer's disease, Parkinson's disease, systemic AA amyloidosis, type 2 diabetes and other amyloidoses is disclosed.

A pharmaceutical agent is disclosed for treating an amyloid disease in a patient, wherein the pharmacological agent comprises a therapeutically effective amount of plant matter from a plant of the family Theraceae, and in particular the genus Camellia. The pharmacological agent is preferably from a plant of the genus Camellia, species sinensis. The pharmacological agent is preferably an extract obtained from Camellia sinensis, the extract being from the dried leaves.

The pharmacological agent preferably has a therapeutically effective amount of standardized green tea leaf extract, or specific catechins, in a dosage in the range of from about 0.1 to about 500 mg/kg of body weight of the patient, and more preferably in the range from about 1.0 to about 100 mg/kg of body weight of the patient.

Preferred pharmaceutical agents have a weight percentage of plant extract in the agent is in the range of from about 70% to about 95%, and may also have a pharmaceutically acceptable carrier, diluent or excipient. The pharmaceutical agent preferably has an amyloid inhibitory activity or efficacy greater than 50%.

Disclosed compositions also have the ability to reduce, eliminate, prevent, inhibit, disrupt, disassemble, or disaggregate amyloid fibril or protein deposits, brain associated amyloid fibril deposits or brain associated amyloid protein deposits, as well as amyloid fibril formation, or age associated amyloid fibril formation, brain associated amyloid fibril formation; it will therefore promote mental acuity, promote mental alertness, provide nutritional support for age or related cognitive or memory decline, promote cognitive well being, support brain function, improve cognitive ability, mental performance or memory, promote concentration and mental sharpness, improve mental vitality, promote greater mental clarity and alertness, improve short term memory, reduce or reverse age associated cognitive or memory decline, support normal brain function, enhance learning or memory; improve concentration, enhance mental performance, reduce mental decline, reduce likelihood of age related brain disorders, and maintain good brain health.

It is anticipated that disclosed compositions also have the ability to reduce, eliminate, prevent, inhibit, disrupt, disassemble or disaggregate amyloid fibril or protein deposits, pancreas associated amyloid fibril or protein deposits, as well as amyloid fibril formation and growth, and pancreas associated amyloid fibril formation and growth; it will therefore support healthy pancreatic function and promote pancreatic function by helping to promote normal insulin function.

Disclosed compositions may also include carriers, diluents and/or excipients commonly used in the pharmaceutical and dietary supplement industries and any such additions as will be known to those skilled in the art are acceptable and may be employed without departing from the scope of the invention.

A method for isolating amyloid inhibitory constituents within green tea, green tea leaves, extracts or derivatives thereof is also disclosed, the method comprising the following steps:
a) extracting the green tea, green tea leaves, extracts or derivatives thereof with water, or with an organic solvent, b) removing insoluble materials, c) evaporation to dryness or lyophilization to obtain powder, d) recovering and redissolving the amyloid inhibitory constituents obtained in the water or organic solvent, and e) injecting and separation by high pressure or low pressure liquid chromatography.

Representative constituents of green tea include catechins, bioflavanoids, flavanols, flavandiols, flavanoids, tannins or derivatives thereof, although for purposes of this disclosure these substance may optionally also be synthetically derived or independently found in other plant sources.

The step of extracting the plant matter with an organic solvent further comprises adding methanol initially to plant materials that are powdered, and the resulting mixture is stirred overnight. The solvent used in the step of extracting amyloid inhibitory ingredients preferably has a polarity ranging from that of water to that of pentanol. The step of removing insoluble materials is preferably affected by centrifuging the extract and collecting the supernatant. The step of concentrating the extract is preferably effected by rotary evaporation or lyophilization (for water extracts). Following the extraction and centrifugation steps, the extraction and centrifugation procedure is preferably repeated 1 to 5 more times and the supernatants are collected.

Following the repeated steps of extraction and concentration, the supernatants are preferably pooled and dried using a rotary evaporator or lyophilization (for water extracts). The dry powder is washed with 4 volumes of petroleum ether (repeated 4 times), followed by centrifugation (each time) at 2,500X g for 20 minutes, and collection of the supernatants and pellets. The collected pellets are air-dried and reextracted with water and centrifuged at 2,500X g for 20 minutes. The collected supernatant (referred to as water extracts) are lyophilized, and the pellets or lyophilized water-extract powder are re-dissolved in acetonitrile/water/trifluoroacetic acid (TFA) for HPLC injection or low pressure chromatography. The dissolved pellet is divided into equal portions and injected into an HPLC. The HPLC preferably contains a 1X25 cm C₁₈ column, though other sizes may be made to serve, and is maintained at 30°C with a flow rate of 2 ml/min. The sample portions injected onto the HPLC are eluted with gradients of A and B, such that 0% B for 5 minutes, 0-15% B from 5-10 minutes, 15-45% B from 10-70 minutes, and 45-100% B from 70-85 minutes; where B=95% acetonitrile with 0.5% acetic acid in distilled water and A=5% acetonitrile with 0.5% acetic acid in distilled water. The eluents from the HPLC are monitored at all wavelengths and 4 ml fractions are collected in a fraction collector and pooled peaks are obtained at various retention times (from 0 through 85 minutes). The fractions obtained may be concentrated by lyophilization after most of the acetonitrile is removed by rotary evaporation.

The concentrated fractions obtained are then tested in relevant *in vitro* assays to identify potent inhibitors of amyloid fibril formation, or disassembly/disruption or disaggregation of pre-formed amyloid fibrils. The amyloid inhibitory ingredients are preferably drawn from the HPLC approximate retention times of 10-70 minutes.

A method is also disclosed for treating an amyloid disease in a patient comprising the step of administering to the patient a therapeutically effective amount of green tea, green tea leaves and extracts or derivatives thereof. The green tea, green tea leaves, and extracts or derivatives thereof, are preferably administered orally or by aerosol spray or in a parentally injectable or infusible form.

In disclosed methods, amyloid formation, deposition, accumulation and/or persistence in a subject is inhibited by administering a therapeutic dose of the invention to the subject. The term "subject" is intended to include living organisms in which amyloidosis can occur. Examples of subjects include humans, monkeys, cows, sheep, goats, dogs, cats, mice, rats and transgenic species thereof. Administration of green tea, green tea leaves and extracts or derivatives thereof, to a subject to be treated can be carried out using known procedures, at dosages and for periods of time effective to inhibit amyloid formation, deposition, accumulation and persistence in the subject. An effective amount of the therapeutic compound necessary to achieve a therapeutic effect may vary according to factors such as the amount of amyloid already deposited at the clinical site in the subject, the age, sex, and weight of the subject, and the ability of the therapeutic compound to inhibit amyloidosis in the subject.

### Representative Method or Process Embodiments

A method of treatment, prevention, or management of an amyloidosis in a mammalian subject susceptible to, or afflicted by, the amyloidosis is presented, the method comprising the step of administering to the subject a therapeutic amount of plant matter from a source of green tea, green tea leaves, standardized green tea extract, or green tea derivative.

A method for the treatment, inhibition, prevention or management of amyloid formation, deposition, accumulation, aggregation and/or persistence in Alzheimer's disease, Parkinson's disease, systemic AA amyloidosis, type 2 diabetes and other amyloidoses in a mammalian subject is presented, the method comprising the step of administering to the subject a therapeutic amount of a substance selected from the group of substances consisting of green tea, green tea leaves, standardized green tea extract, green tea derivative, catechins, bioflavanoids, flavanols, flavandiols, flavanoids, tannins or derivatives thereof.

The substance is preferably a catechin selected from the group of catechins consisting of catechin, epicatechin, gallocatechin gallate, epigallocatechin gallate, epigallocatechin, and epicatechin gallate, or a derivative of one of the above group.

In any of these embodiments, within the step of administering plant matter, a therapeutic quantity of one or more plant materials selected from the group of plants consisting of, and commonly known as, Cat's claw, ginkgo biloba, rosemary, gotu kola, bacopin, and ginseng may also optionally be administered.

A method for the treatment, inhibition, prevention or management of alpha-synuclein fibril formation, deposition, accumulation, aggregation and/or persistence in Parkinson's disease, Lewy body disease, or multiple system atrophy, in a mammalian subject is presented, the method comprising the step of administering to the subject a therapeutic amount of a substance selected from the group of substances consisting of green tea, green tea leaves, standardized green tea extract, green tea derivative, catechins, bioflavanoids, flavanols, flavandiols, flavanoids, tannins or derivatives thereof.

The substance is preferably a catechin selected from the group of catechins consisting of catechin, epicatechin, gallocatechin gallate, epigallocatechin gallate, epigallocatechin, and epicatechin gallate, or a derivative of one of the above group.

A method for promoting mental alertness in a patient is presented, the method comprising the step of administering to the patient a therapeutically effective amount of plant matter from a plant of the family Theaceae, and preferably from a plant of the genus Camellia, species sinensis. This method may also be used for inhibiting the formation of brain amyloid deposits.

A method for promoting, maintaining or enhancing in a patient one or more of the mental or cognitive qualities selected from the group of mental or cognitive qualities consisting of mental acuity, mental alertness, cognitive well being, normal brain function, cognitive ability, mental performance, memory, concentration, mental sharpness, mental clarity, short term memory, normal brain function, and learning is presented, the method comprising the step of administering to the patient a therapeutically effective amount of plant matter from a plant of the genus Camellia, species sinensis.

A method for providing, supporting or improving in a patient one or more of the mental or cognitive qualities selected from the group of mental or cognitive qualities consisting of normal brain function, cognitive ability, and concentration is presented, the method comprising the step of administering to the patient a therapeutically effective amount of plant matter from a plant of the genus Camellia, species sinensis.

A method for reducing in a patient one or more of the mental or cognitive effects selected from the group of mental or cognitive effects consisting of, age associated cognitive or memory decline, mental decline, and likelihood of age related brain or cognitive disorders is presented, the method comprising the step of administering to the patient a therapeutically effective amount of plant matter from a plant of the genus Camellia, species sinensis.

A method for reducing, disrupting, dissolving, inhibiting, eliminating or preventing in a patient one or more conditions involving the brain selected from the group of conditions involving the brain consisting of amyloid fibril deposits, amyloid protein deposits, brain associated amyloid fibril deposits, brain associated amyloid protein deposits, amyloid fibril formation and growth, age associated amyloid fibril formation and growth, brain associated amyloid fibril formation and growth is presented, the method comprising the step of administering to the patient a therapeutically effective amount of plant matter from a plant of the genus Camellia, species sinensis.

A method for promoting or supporting healthy pancreatic function in a patient, by helping to promote normal insulin function is presented, the method comprising the step of administering to the patient a therapeutically effective amount of plant matter from a plant of the genus Camellia, species sinensis.

A method for reducing, disrupting, dissolving, inhibiting, eliminating or preventing in a patient one or more conditions involving the pancreas selected from the group of conditions involving the pancreas consisting of amyloid fibril deposits, amyloid protein deposits, pancreas associated amyloid fibril deposits, pancreas associated amyloid protein deposits, amyloid fibril formation and growth, pancreas associated amyloid fibril formation and growth is presented, the method comprising the step of administering to the patient a therapeutically effective amount of plant matter from a plant of the genus Camellia, species sinensis.

### Representative Use And/or Composition/Agent Embodiments

The use of a source of green tea, green tea leaves or standardized green tea leaf extract or derivatives thereof in the preparation of a pharmaceutical composition or dietary supplement for the treatment, prevention and or management of an amyloidosis in a mammalian subject susceptible to, or afflicted by, the amyloidosis is presented.

The use of a source of green tea, green tea leaves, standardized green tea leaf extract or derivatives thereof in the preparation of a pharmaceutical composition or dietary supplement for inhibiting amyloid fibril formation, deposition, accumulation, or persistence or causing dissolution/disruption or disaggregation of pre-formed amyloid fibrils is presented.

A pharmaceutical composition or dietary supplement for the treatment, prevention, or management of amyloidosis in a mammalian subject susceptible to, or afflicted by, the amyloidosis is presented, the composition comprising a source of green tea, green tea leaves or standardized green tea leaf extract or derivatives thereof, and, if desired, a pharmaceutically or dietarily acceptable carrier, diluent or excipient.

A pharmaceutical composition or dietary supplement for inhibiting amyloid fibril formation, deposition, accumulation, or persistence or causing dissolution/ disruption and or disaggregation of pre-formed amyloid fibrils is presented, the composition comprising a source of green tea, green tea leaves or standardized green tea leaf extract or derivatives thereof and, if desired, a pharmaceutically or dietarily acceptable carrier, diluent or excipient.

The use of catechins, bioflavanoids, flavanols, flavandiols, flavanoids, tannins or derivatives thereof for the treatment, prevention or management of amyloid formation, deposition, accumulation and/or persistence in Alzheimer's disease, Parkinson's disease, systemic AA amyloidosis, type 2 diabetes and other amyloidoses, in a mammalian subject susceptible to the amyloidosis is presented.

A pharmaceutical composition or dietary supplement for the treatment, prevention or management of amyloid formation, deposition, accumulation and/or persistence in Alzheimer's disease, Parkinson's disease, systemic AA amyloidosis, type 2 diabetes and other amyloidoses in a mammalian subject susceptible to such amyloid condition is presented, the composition comprising catechins, bioflavanoids, flavanols, flavandiols, flavanoids, tannins or derivatives thereof and, if desired, a pharmaceutically or dietarily acceptable carrier, diluent or excipient.

A pharmaceutical composition or dietary supplement for the treatment, inhibition, prevention or management of alpha-synuclein fibril formation, deposition, accumulation, aggregation and/or persistence in Parkinson's disease, Lewy body disease, or multiple system atrophy in a mammalian subject is presented, the composition comprising a substance selected from the group of substances consisting of green tea, green tea leaves, standardized green tea extract, green tea derivative, catechins, bioflavanoids, flavanols, flavandiols, flavanoids, tannins or derivatives thereof.

The use of a source of green tea, green tea leaves or standardized green tea leaf extract or derivatives thereof in the preparation of a pharmaceutical composition or dietary supplement for providing, supporting or improving in a subject one or more of the mental or cognitive qualities is presented.

The use of a source of green tea, green tea leaves or standardized green tea leaf extract or derivatives thereof in the preparation of a pharmaceutical composition or dietary supplement for promoting or supporting healthy pancreatic function in a subject is presented.

A pharmaceutical composition or dietary supplement for providing, supporting or improving in a subject one or more of the mental or cognitive qualities which comprises a source of green tea, green tea leaves or standardized green tea leaf extract or derivatives thereof is presented.

A pharmaceutical composition or dietary supplement for promoting or supporting healthy pancreatic function in a subject which comprises a source of green tea, green tea leaves or standardized green tea leaf extract or derivatives thereof is presented.

A pharmacological agent for promoting, maintaining or enhancing in a patient one or more of the mental or cognitive qualities selected from the group of mental or cognitive qualities consisting of mental acuity, mental alertness, cognitive well being, normal brain function, cognitive ability, mental performance, memory, concentration, mental sharpness, mental vitality, mental clarity, short-term memory, normal brain function, and learning, and good brain health is presented, wherein the pharmacological agent comprises a therapeutically effective amount of plant matter from a plant of the genus Camellia, species sinensis.

A pharmacological agent for providing, supporting or improving in a patient one or more of the mental or cognitive qualities selected from the group of mental or cognitive qualities consisting of nutritional support for age related cognitive or memory decline, normal brain function, cognitive ability, and concentration is presented, wherein the pharmacological agent comprises a therapeutically effective amount of plant matter from a plant of the genus Camellia, species sinensis.

A pharmacological agent for reducing in a patient one or more of the mental or cognitive effects selected from the group of mental or cognitive effects consisting of, age associated cognitive or memory decline, mental decline, and likelihood of age related brain or cognitive disorders is presented, wherein the pharmacological agent comprises a therapeutically effective amount of plant matter from a plant of the genus Camellia, species sinensis.

A pharmacological agent for reducing, disrupting, dissolving, inhibiting or preventing in a patient one or more conditions involving the brain selected from the group of conditions involving the brain consisting of amyloid fibril deposits, amyloid protein deposits, brain associated amyloid fibril deposits, Aß brain deposits, brain associated Aß deposits, brain associated amyloid protein deposits, brain amyloid deposits, amyloid fibril formation and growth, age associated amyloid fibril formation and growth is presented, wherein the pharmacological agent comprises a therapeutically effective amount of plant matter from a plant of the genus Camellia, species sinensis.

A pharmacological agent for promoting or supporting healthy pancreatic function in a patient, by helping to promote normal insulin function is presented, wherein the pharmacological agent comprises a therapeutically effective amount of plant matter from a plant of the genus Camellia, species sinensis.

A pharmacological agent for reducing, disrupting, dissolving, inhibiting or eliminating or preventing in a patient one or more conditions involving the pancreas selected from the group of conditions involving the pancreas consisting of amyloid fibril deposits, amyloid protein deposits, pancreas associated amyloid fibril deposits, amylin deposits, islet amyloid polypeptide deposits, pancreas associated amyloid protein deposits, amyloid fibril formation and growth, pancreas associated amyloid fibril formation and growth is presented, wherein the pharmacological agents comprises a therapeutically effective amount of plant matter from a plant of the genus Camellia, species sinensis.

For a more detailed discussion of and background information pertaining to amyloid and amyloidosis, Alzheimer's disease and the aging population, amyloid as a therapeutic target for Alzheimer's disease and Parkinson's disease and alpha-synuclein fibril formation, see our co-pending US patent application 20030017998 and issued patents 6,037,327, 6,264,994 and 6,346,280.

These and otherfeatures and advantages will become more fully apparent when the following detailed description is read in conjunction with the accompanying figures.

### BRIEF DESCRIPTION OF THE DRAWINGS

**FIGURE 1** is a set of structures for catechin derivatives.
**FIGURE 2** is a black and white graph of a Thioflavin T fluorometry assay utilized to determine the dose-dependent effects of standardized green tea leaf extract on inhibition of Alzheimer's Aß 1-40 amyloid fibril formation.
**FIGURE 3** is a black and white graph of a Thioflavin T fluorometry assay utilized to determine the dose-dependent effects of standardized green tea leaf extract on disassembly/disruption of pre-formed Alzheimer's Aß 1-42 amyloid fibrils.
**FIGURE 4** is a black and white graph of a Congo red-Aß spectrophotometric assay to determine the effects of standardized green tea leaf extract (from 2 commercial sources) on disaggregation of pre-formed Alzheimer's Aß 1-42 fibrils.
**FIGURE 5** is a black and white graph of a Thioflavin T fluorometry assay utilized to determine the dose-dependent effects of different commercially available catechins on inhibition of Alzheimer's Aβ 1-40 amyloid fibril formation.
**FIGURE 6** is a color photomicrograph demonstrating the inhibition of Alzheimer's Aβ 1-40 fibril formation by epicatechin.
**FIGURE 7** is a black and white graph of a Thioflavin T fluorometry assay utilized to determine the dose-dependent effects of commercially available catechins on disassembly/disruption of pre-formed Alzheimer Aβ 1-42 amyloid fibrils.
**FIGURE 8** is a black and white graph of a Thioflavin T fluorometry assay utilized to determine the dose-dependent effects of different commercially available catechins on inhibition of Parkinson's disease NAC (also referred to as "NAC-P") fibril formation.
**FIGURE 9** shows black and white photomicrogrpahs of amyloid deposition in spleen in a mouse model of experimental AA amyloidosis,
**FIGURE 10** shows black and white photomicrographs of amyloid deposition in liver in a mouse model of experimental AA amyloidosis.
**FIGURE 11** is a graph demonstrating the effect of specific polyphenolic compounds on disruption/disassembly of pre-formed amyloid fibrils consisting of islet amyloid polypeptide (IAPP).
**FIGURE 12** is a graph of circular dichroism spectroscopy demonstrating the disruption of ß-sheet structure in Aß 1-42 amyloid fibrils by epicatechin.
**FIGURE 13** is a graph demonstrating the inhibition of splenic amyloid by epicatechin, but not catechin hydrate, in an experimental mouse model of systemic AA amyloidosis.
**FIGURE 14** shows black and white photomicrographs of inhibition of splenic AA amyloid deposition by epicatechin in a mouse model of systemic AA amyloidosis.
**FIGURE 15** illustrates the structures of some of the polyhydroxylated aromatic containing-compounds conceived to have anti-amyloid activity.

### BEST MODE OF CARRYING OUT THE INVENTION

Turning now to the Drawings and Examples, the invention will be described in preferred embodiments by detailed reference to them.

### Some Definitions

"Pharmaceutically acceptable analogs and derivatives." Pharmaceutically acceptable analogs and derivatives of a claimed compound include various R- group type substitutions, and any other derivative structural modifications not affecting the disclosed efficacy of these compounds.

"Percentage Purity." Disclosed compositions contain one or more catechins or green tea derivatives, each catechin or green tea derivative present in the composition in a proportion percentage or percentage purity that "significantly exceeds" a proportion percentage of the same substance's natural presence in a plant, or in an extract from the plant. For example, suppose that a particular catechin is present in a plant in a percentage by weight of 0.01 percent, and is present in an extract of the plant in a percentage by weight of 1.0 percent. In a disclosed composition then, the same catechin is present in the composition in a percentage by weight that is significantly greater than 0.01 percent or 1.0 percent, say 10 percent. Other proportionalities along this line may be applied as well, such as percentage composition or percentage presence by volume, or percent purity.

By way of further example, without limiting the scope of invention to this or other examples, a catechin is present in a tablet to be delivered orally in accordance with the disclosure herein. The catechin is an isolated catechin present in a percentage purity of 98.5% (that is, the catechin is 98.5% pure, as measured by conventional purity indicia, such as for example the characteristic single sharp peak band on an HPLC). The particular catechin is however only a 15% ingredient by weight in the tablet. The catechin is known to be present in a plant in a dry weight percentage of 0.06, while certain extracts of the same plant are known to contain up to 0.75 percent dry weight percent of the same catechin. In this example, the catechin is proportionally more present in the tablet than in the extract by a ratio of 20:1, and this is one measure of significantly exceeding the natural proportion percentage presence in a plant or extract of the plant.

In general, a catechin present in a therapeutically administered dose form that has a percentage of the catechin (by weight, dry weight, volume, or purity) that is 10 times (or more) greater than the natural percentage presence of the same catechin in a plant is a percentage that ""significantly exceeds" the natural percentage presence of the catechin in the plant. When speaking of extracts of a plant in this context, it should be noted that only conventional or natural extracts are to be considered (juices, concentrates and the like, or extracts known and used for other purposes), not new extracts prepared after the priority date of this disclosure the effect of which is to concentrate the particular catechin so as to negative a finding of ""significantly exceeding", as just defined. It should also be noted that in some cases, a finding of "significantly exceeding" may be justified with a ratio of as little as 2:1, but more preferably as great as 50:1 to 100:1.

In the example of a single catechin compound with an excipient to make up the composition, it may be convenient merely to note and compare the percent purity of the compound in the composition, rather than its overall weight percentage, for purposes of the "significantly exceeding" standard, as claimed. In the case of mixtures of catechins it can be appropriate to view a combined percentage composition of the mixed catechins in the therapeutic dosage and compare that figure to a combined percentage presence of the same catechin's in the natural plant or extract.

In any event, the purpose of the disclosed standard of measurement is to set forth a fair margin by which a claimed composition exceeds reading on the active ingredients' natural occurrence in plants and conventional extracts of plants.

Preferred compositions will contain catechin that is at least substantially pure. Catechin that is in substantially pure isolated or synthetic form may be advantageously employed as well. In general "pure" means better than 95% pure, and "substantially pure" catechin means a catechin purified by extraction or other known means or means disclosed herein such that the catechin is present in the therapeutic dosage with only those impurities that can not readily nor reasonably be removed by the extraction or purification processes. "Isolated" means that the catechin in question is not accompanied in the therapeutic form by significant quantities of other catechins. An "isolated pure" compound is a compound in isolated purified form such as is conventional for active ingredients in the pharmaceutical industry.

### A Cell Culture Method to Generate AA Amyloid

A number of previous studies indicate that AA amyloid formation can be achieved in cell culture model systems (Shirahama et al, Lab, Invest, 62:61-68, 1990; Palm et al, APMIS 105:603-608, 1997; Kluve-Beckerman et al, Am. J. Path. 155: 123. 133, 1999). Probably, the best cell culture system in which to produce fibrillar AA amyloid protein was described by Kluve-Beckerman et al (Am. J. Path. 155:123-133, 1999). In this *in vitro* system, murine peritoneal macrophage cells (isolated from the peritoneal cavity of normal C57BL/6 mice) were maintained, to which recombinant SAA2 (rSAA2) in neutral pH medium, and/or amyloid enhancing factor (AEF)(discussed below) were added. The medium containing recombinant SAA2 was used at a concentration typical to that seen in acute phase serum (i.e. 140µg/ml). Within 24-48 hours after treatment, cultures developed foci of AA amyloid deposits, which enlarged and became increasingly congophilic and birefringent (following Congo red staining as viewed under polarized light). This study demonstrated that SAA2 was cleaved by the macrophages to form AA amyloid protein (~8.5 kDa fragment) in culture, which was fibrillar (as demonstrated by positive Congo red birefringence and Thioflavin S fluorescence). The peritoneal cells were found to be viable (as determined by exclusion of trypan blue staining) and maintained phagocytosis activity (as shown by the phagocytosis of latex beads). In addition, cultures of peritoneal cells obtained from nude mice demonstrated similar amyloid formation and deposition in culture, indicating that T lymphocytes were not essential for amyloid fibril formation.

Although AEF was not required, its addition to the culture medium resulted in larger and more numerous amyloid deposits. A greater amount of fibrillar AA amyloid deposition in culture was also observed when cultures were treated with pepstatin, an aspartic protease inhibitor (Kluve-Beckerman et al, Am. J. Path. 155: 123-133, 1999). These cultures also maintained viability similar to those of untreated cultures, even as long as 24 days. Pepstatin-treated cultures developed large areas of dense, localized masses of amyloid associated with cell clusters.

This cell culture system is both easy to employ and amenable to manipulation for the development of AA amyloid deposition and persistence *in vitro.*

### A Relevant Mouse Model of Experimental AA Amyloidosis

A number of good experimental animal models exist for the production of systemic AA amyloidosis *in vivo*. Early studies involving repeated injections of azocasein (derivative of casein) demonstrated the accumulation of AA amyloid deposits in spleen, liver and kidney occurred within 14-21 days following treatment (Janigan and Druet, Am. J. Path 48:1013-1024, 1966). In this model, the injection of the azocasein resulted in a sterile abscess, which caused an inflammatory reaction inducing elevated serum levels of SAA in the mouse (Kindy and DeBeer, Methods Enzym. 309:701-716, 1999). The AA amyloid deposits were fibrillar as indicated by their positive staining with Congo red (i.e. red/green birefringence as viewed under polarized light).

In more recent animal models, systemic AA amyloid deposition can be induced rapidly in CBA/J mice following a single subcutaneous injection of 2% silver nitrate (the inflammatory stimulus) plus a single intravenous injection of amyloid enhancing factor (AEF)(discussed below). In this model, AA amyloid deposition occurs first in the spleen (by 24-48 hours), then in liver (by 3-4 days), followed by kidney (5-7 days)(Axelrad et al, Am. J. Path. 78:277-284, 1975; Kisilevsky et al, Lab. Invest. 37:544-553, 1977). We have previously utilized this model extensively to study the development of AA amyloidosis and the role that specific proteoglycans/glycosaminoglycans play in amyloid formation (Kisilevsky et al, Appl. Path. 2:308-315,1984; Snow et al, Lab. Invest. 53:37-44,1985; Lab. Invest. 56:665-675, 1987; Lab. Invest, 57:687-698, 1987; J. Histochem. Cytochem. 39:1321-1330, 1991). This relevant experimental model is already in use and is specifically utilized to identify and test the efficacy of potential AA amyloid therapeutics, as described below.

The experimental CBA/J mouse model (as described above) is an ideal system to test the efficacy of potential AA amyloid therapeutics since following an inflammatory stimulus (i.e. silver nitrate) and AEF, 100% of test animals will deposit amyloid in tissues within the span of a few days. Whereas only a small percentage of patients with precursor proteins actually develop amyloid, all animals in this experimental model do so. The inflammatory reaction in CBA/J mice, unlike rheumatoid arthritis or osteomyelitis, for example, results in the appearance of all the prerequisites for amyloid deposition to occur.

AEF dramatically shortens the time necessary for the experimental induction of AA amyloid deposition in CBA/J mice and can be demonstrated in tissues 24 - 48 hours before the appearance of amyloid (Axelrad et al, Lab. Invest. 47:139-146, 1982). Although the actual identity of AEF has not been fully resolved, it is believed to be generally protein in nature (Axelrad et al, Lab. Invest. 47:139-146, 1982), or consists of a small nidus of amyloid fibril-like material that contains abundant β-sheet (Kisilevsky et al, Amyloid:Int. J. Exp. Clin. Invest. 6:98-106, 1999). AEF is usually prepared from amyloid-laden spleens and/or livers of animals previously induced for AA amyloid accumulation following daily azocasein injections or single injections of AEF and silver nitrate. These amyloidotic tissues are homogenized in phosphate-buffered saline (PBS) containing protease inhibitors, and the pellet is re-homogenized a number of times. The second through fourth supernatants (obtained following water extractions) generally contain potent AEF activity that is used to rapidly induce AA deposition in animals.

Discovery and identification of new compounds or agents as potential therapeutic agents to arrest amyloid fibrilar amyloid formation, deposition, accumulation and/or persistence that occurs in Alzheimer's disease, systemic AA amyloidosis, type 2 diabetes, and other amyloidoses, and fibrillogenesis in Lewy body disease, Parkinson's disease, and multiple system atrophy, are desperately sought. Use of Catechins, Bioflavanoids, Flavanols, Flavandiols, Flavanoids, Tannins (or derivatives of any of these) from Green Tea, Green Tea Leaves, Green Tea Extracts or Green Tea Derivatives, or from other Natural or Synthetic Sources

Green tea, and other natural sources such as black tea and wine, are known to contain catechins, bioflavanoids, flavanols, flavandiols, flavanoids, tannins or derivatives thereof. See Sugita-Konishi et al, "Epigallocatechin gallate and gallocatechin gallate in green tea catechins inhibit extracellular release of Vero toxin from enterohemorrhagic E. coli", Biochemica et Biophysica Acta, 1472, 42-50 (1999), and Fernandez et al, "HPLC determination of catechins and caffeine in tea", Analyst 125: 421-425 (2000). These constituents are now believed by us to play a significant role in the surprisingly beneficial effects of green tea in amyloidoses as discussed below.

In particular the catechins of the group consisting of catechin, epicatechin, gallocatechin gallate, epigallocatechin gallate, epigallocatechin, and/or epicatechin gallate or a derivative of one of the above group, are now believed by us to be effective, either alone or in combination with other amyloid inhibitory ingredients such any plant matter from the group of plants consisting of, and commonly known as, Cat's claw, ginkgo biloba, rosemary, gotu kola, bacopin, and ginseng, to achieve any or all of the beneficial effects described below.

General structures for representative catechins are presented in Figure 1. It is expected that pharmaceutically acceptable analogs and derivatives of these catechin structures, such as for example various R- group type substitutions, and other derivative structural modifications not affecting the disclosed efficacy of these compounds may be made without affecting the scope of the appended claims.

### Use of Standardized Green Tea Leaf Extract to Inhibit Amyloidosis

The Examples illustrated below all serve well to establish that, at least *in vitro,* green tea, green tea leaves and extracts or derivatives thereof, have the ability to inhibit the formation of brain amyloid deposits that occur during normal aging and in a variety of brain disorders including Alzheimer's disease. In addition, it is known that patients who accumulate brain amyloid deposits eventually lose cognitive ability and memory function and sustain a marked reduction in mental clarity in general. Therefore it follows that inhibition of such brain amyloid deposits will at the least promote mental alertness in such patients.

The Examples also establish that again, at least *in vitro,* green tea, green tea leaves and extracts or derivative thereof, have the ability to reduce, eliminate, prevent, inhibit, disrupt/dissolve, or disaggregate amyloid fibril or protein deposits, as well as amyloid fibril formation, or age associated amyloid fibril formation, and brain associated amyloid fibril formation. In addition, it is known that patients who accumulate amyloid fibril or protein deposits, brain associated fibril deposits or brain associated amyloid protein deposits, or who display symptoms of amyloid fibril formation and growth or age associated amyloid fibril formation and growth, brain associated amyloid fibril formation and growth, in general will eventually lose mental acuity, mental alertness, concentration, cognitive well being, or some measure of brain function or cognitive ability, mental performance or memory, or concentration and mental sharpness, or mental vitality, or mental clarity and alertness, short term memory, or some of the ability to learn and remember. It is also known that such patients are subject to age associated or related cognitive or memory decline, or will sustain a marked reduction in mental clarity.

It follows then that inhibition, reduction, elimination, prevention, disruption, disassembly or disaggregation of such amyloid fibril or protein deposits, brain associated amyloid fibril deposits or brain associated amyloid protein deposits, or amyloid fibril formation and growth, or age associated amyloid fibril formation and growth; will improve mental acuity, promote mental alertness, provide nutritional support for age related cognitive or memory decline, promote cognitive well being, support brain function, improve cognitive ability, mental performance or memory, promote concentration and mental sharpness, improve mental vitality, promote greater mental clarity and alertness, improve short term memory, reduce or reverse age associated cognitive or memory decline, support normal brain function, enhance learning or memory; improve concentration, enhance mental performance, reduce mental decline, reduce likelihood of age related brain disorders, and maintain good brain health, in such patients.

The Examples further suggest that green tea, green tea leaves and extracts or derivatives thereof, should have the ability to reduce, eliminate, prevent, inhibit, disrupt, dissolve, disassemble, disaggregate amyloid fibril or protein deposits, pancreas associated amyloid fibril or protein deposits, as well as amyloid fibril formation and growth, and pancreas associated amyloid fibril formation and growth. In addition, it is known that patients who accumulate amyloid fibril or protein deposits, pancreas associated amyloid fibril or protein deposits, or who display symptoms of amyloid fibril formation and growth, pancreas associated amyloid fibril formation and growth, in general lose healthy pancreatic function or sustain a reduction in normal insulin function, leading to loss or reduction of pancreatic function. It there follows that inhibition, reduction, elimination, prevention, disruption, disassembly, dissolution or disaggregation of such amyloid fibril or protein deposits, pancreas associated amyloid fibril or protein deposits, or amyloid fibril formation and growth, pancreas associated amyloid fibril formation and growth, will support healthy pancreatic function and promote pancreatic function by helping to promote normal insulin function in such patients.

Recent studies, published after the priority date of this application, now further support these experimental results. See Hasegawa, "Preventive effect of Japanese green tea against cognitive impairment in the elderly", posters 42 and 755, proceedings of World Alzheimer Congress 2000, in Neurobiology of Aging, 21:18 (2000), reporting statistically significant relationship between increased drinking of green tea and higher cognitive levels.

### EXAMPLES

The following examples are put forth so as to provide those with ordinary skill in the art with the disclosure and description and use of commercially available green tea extract and catechins which surprisingly are shown to cause an inhibition, disassembly/disruption and/or disaggregation of Alzheimer's disease Aß-containing fibrils, and Parkinson's disease NAC fibrils. However, it should not be construed that the invention is limited to these specific examples.

### Example 1

### Standardized Green Tea Leaf Extract is a Potent Inhibitor of Alzheimer's Aß (1-40) Amyloid Fibril Formation

A previously described method of measuring amyloid fibril formation utilizing Thioflavin T fluorometry (H Naiki et al, Lab. Invest. 65:104-110, 1991; H Levine III, Protein Sci. 2:404-410, 1993; H Levine III, Amyloid: Int. J. Exp. Clin. Invest. 2:1-6, 1995; H Naiki and K. Nakakuki, Lab. Invest. 74:374-383, 1996) was employed initially to identify whether standardized green tea leaf extract was capable of inhibiting Alzheimer's Aß amyloid fibril formation. Using this sensitive assay, any decreases or increases in fluorescence was previously shown to correlate with a decrease or increase in the amount of amyloid fibrils (H Naiki et al, Lab. Invest. 65:104-110, 1991; H Levine III, Protein Sci. 2:404-410,1993; H Levine III, Amyloid: Int. J. Exp. Clin. Invest. 2:1-6, 1995; H Naiki and K. Nakakuki, Lab. Invest. 74:374-383, 1996), allowing one to determine the effects of potential inhibitors and/or enhancers of amyloid fibril formation.

In a first study, the effects of standardized green tea extract as a potent Alzheimer's disease amyloid inhibitory agent on Alzheimer's Aß (1-40) fibril formation was assessed by Thioflavin T fluorometry. Thioflavin T is known to bind to fibrillar amyloid proteins, and an increase in fluorescence correlates with an increase in amyloid fibril formation, whereas a decrease in fluorescence correlates with a decrease in amyloid fibril formation. The Alzheimer's Aß protein (1-40) when incubated at 37°C tends to spontaneously form amyloid fibrils that increase in quantity over time. In this study, we tested for standardized green tea extract to inhibit the Alzheimer's amyloid Aß protein from forming fibrils over a 1 week period. For this study, 25 µM of Aß (1-40)(Bachem Inc., Torrance, CA, USA; Lot #WM365) was incubated in microcentrifuge tubes at 37°C for 1 week (in triplicate), either alone, or in the presence of 10 µg/ml, 50µg/ml or 100µg/ml of standardized green tea extract in 150 mM Tris HCl, 10 mM NaCl, pH 7.0 (TBS). For this study, the powder within one gelatin capsule of standardized green tea extract obtained from a commercial source (Nature's Resource, Mission Hills, CA) was extracted in 1 ml of distilled water and pelleted using a microcentrifuge (for 10 minutes at 2,500X g). The supernatant was then taken and lyophilized. A 1 mg/ml working solution for use in the *in vitro* assays described below was then made using distilled water. The commercial green tea leaf extracts are usually standardized to 50% polyphenols.

To assess the effects of standardized green tea extract on Aß (1-40) fibril formation, 50 µl aliquots were taken from each tube for analysis at 1 hr, 1 day, 3 days, and 1 week. For each determination described above, following each incubation period, 50µl of Aß +/- standardized green tea extract were added to 1.2ml of 100µM Thioflavin T (Sigma Chemical Co., St. Louis, MO) in 50mM NaPO₄ (pH 6.0). Studies indicated that increasing concentrations of fibrillar Aß gave a proportional increase in fluorescence in the presence of 100µM Thioflavin T, ruling out the presence of any disproportionate inner filter effects in these studies. Fluorescence emission at 482 nm was measured on a Turner instrument-model 450 fluorometer at an excitation wavelength of 450 nm. For each determination, the fluorometer was calibrated by zeroing in the presence of the Thioflavin T reagent alone, and by setting the 50 ng/ml riboflavin (Sigma Chemical Co., St. Louis, Mo) in the Thioflavin T reagent to 1800 fluorescence units. All fluorescence determinations were based on these references and any fluorescence given off by any of the compounds in the presence of the Thioflavin T reagent was always subtracted from all pertinent readings.

For all fibrillogenesis studies utilizing Thioflavin T fluorometry, as disclosed herein, comparisons of amyloid protein in the presence or absence of standardized green tea extract were based on paired Student's t tests with data shown as mean +/standard deviation. Significance was reported at the 95% (p< 0.05), 99% (p<0.01) and 99.5% (p<0.005) confidence levels.

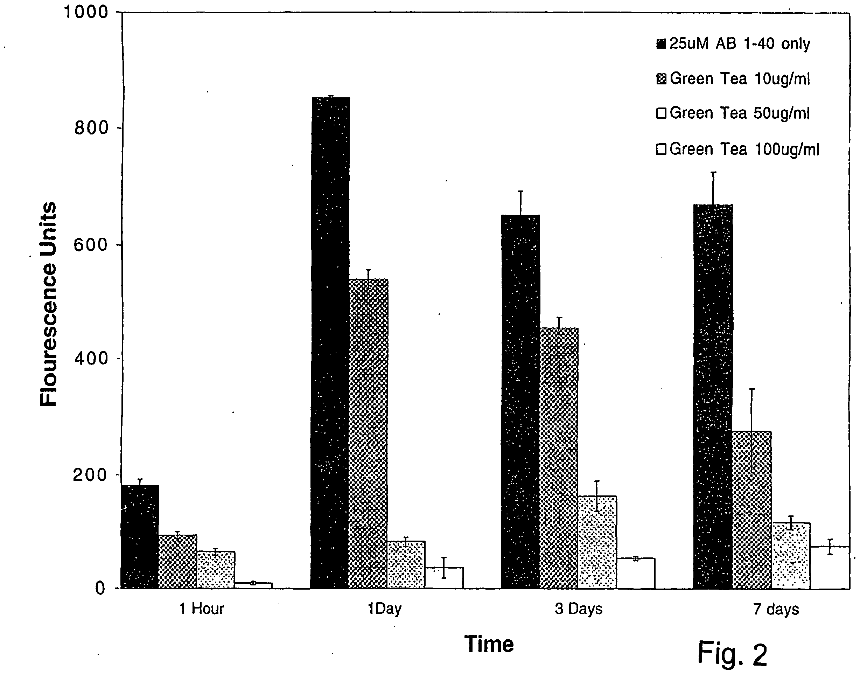

As shown in Figure 2, the effects standardized green tea extract on Alzheimer's Aß (1-40) amyloid fibril formation was evaluated over a 1-week incubation period. Freshly suspended Aß (1-40) alone, following a 1-hour incubation at 37°C, demonstrated an initial fluorescence of 183 +/- 10 fluorescence units. During the 1-week incubation period, there was a gradual increase in the fluorescence of Aß (1-40) alone, increasing approximately 4-fold from 1 hour to 1 day, with a peak fluorescence of 852 +/- 3 fluorescence units observed at 1 day (Figure 2), consistent with previous studies (Castillo et al, J. Neurochem. 69:2452-2465, 1997). Standardized green tea extract significantly inhibited Aß (1-40) amyloid fibril formation in a dose-dependent manner as early as 1 hour of incubation (Figure 2). Significant inhibition (p<0.005) by standardized green tea leaf extract on Aß 1-40 amyloid fibril formation was observed at all time points including 1 hour, 1 day, 3 days and 1 week (Figure 2). At 1 hour, 10µg/ml, 50µg/ml and 100 µg/ml of standardized green tea leaf extract significantly (p<0.005) inhibited Aß 1-40 fibril formation by 50.3 +/- 3.3%, 66.1 +/- 3.3%, and 95.1+/- 1.6%, respectively. At 1 day, 10µg/ml, 50µg/ml and 100 µg/ml of standardized green tea leaf extract significantly (p<0.005) inhibited Aß 1-40 fibril formation by 36.7+/- 2.0%, 90.7+/-1.0%, and 95.9+/-2.0%, respectively. By 1 week, 10µg/ml, 50µg/ml and 100 µg/ml of standardized green tea leaf extract significantly (p<0.005) inhibited Aß 1-40 fibril formation by 58.9+/-10.7%, 83.0+/-1.8%, and 89.3 +/- 2.1%, respectively. This initial data indicated that standardized green tea extract and at least one of its catechin, bioflavanoid, flavanol, flavandiol, flavanoid or tannin active constituents was a potent inhibitor of Alzheimer's amyloid fibril formation.

### Example 2

### Disassembly/Disruption of Alzheimer's Disease Aß 1-42 Amyloid Fibrils by Standardized Green Tea Leaf Extract

In the next study, standardized green tea leaf extract was tested for its ability to cause a disassembly/disruption of pre-formed Alzheimer's disease amyloid fibrils containing Aß 1-42. This type of activity would be important for any potential anti-amyloid compound which can be used in patients who already have substantial amyloid deposition in organs and/or tissues. For example, Alzheimer's disease patients in mid-to-late stage disease have abundant Aß-containing amyloid deposits in their brains as part of both neuritic plaques and cerebrovascular amyloid deposits. A compound capable of causing disassembly/disruption of pre-existing amyloid deposits would be advantageous for use in these patients who are at latter stages of the disease process.

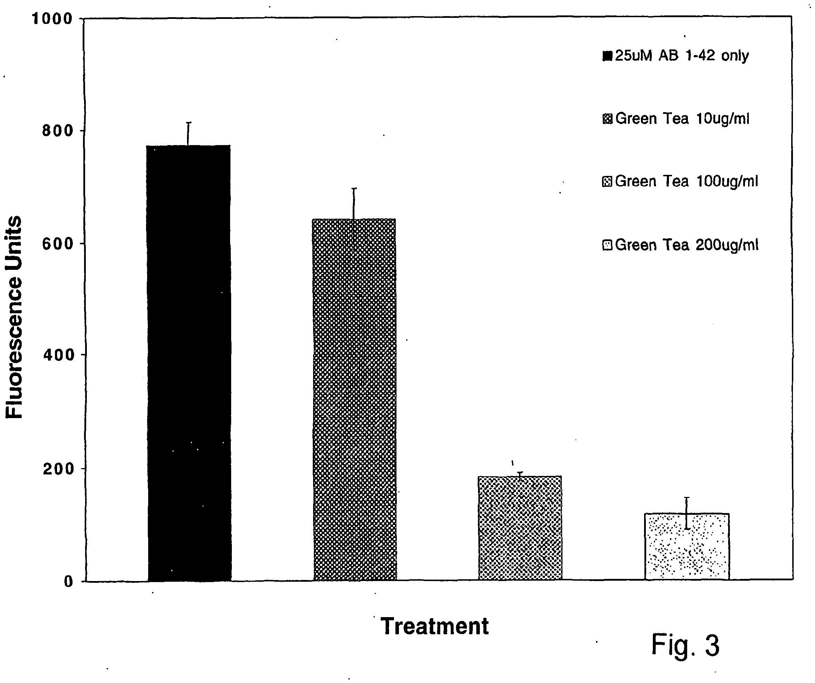

For this study, 1 mg of Aß 1-42 (Bachem Inc., Torrance, CA, USA; Lot#516817) was dissolved in 1.0 ml of double distilled water (1 mg/ml solution). 25 µM of Aß 1-42 was then incubated overnight (~18 hours) at 37°C, in the absence or presence of 10µg/ml, 100µg/ml or 200µg/ml of standardized green tea leaf extract in the presence of 150 mM Tris HCl, 10 mM NaCl (pH 7.0) with 0.02% sodium azide. In these studies (see results described below and in Figure 3), the Aß 1-42:green tea extract weight ratio was 1:0.1, 1:1 and 1:2, respectively.

For this study, the powder within one gelatin capsule of standardized green tea extract obtained from a commercial source (Nature's Resource, Mission Hills, CA) was extracted in 1 ml of distilled water and pelleted using a microcentrifuge (for 10mins at 2,500X g). The supernatant was then taken and lyophilized. A 1 mg/ml working solution for use in the *in vitro* assays described below was then made using distilled water. The commercial green tea leaf extracts are usually standardized to 50% polyphenols.

A previously described method of measuring amyloid fibril formation utilizing Thioflavin T fluorometry (H Naiki et al, Lab. Invest. 65:104-110, 1991; H Levine III, Protein Sci. 2:404-410, 1993; H Levine III, Amyloid: Int. J. Exp. Clin. Invest. 2:1-6, 1995; H Naiki and K. Nakakuki, Lab. Invest. 74:374-383, 1996) was employed to assess whether standardized green tea leaf extract is capable of causing a disassembly/disruption of Alzheimer's Aß 1-42 amyloid fibrils. Thioflavin T is known to bind to fibrillar amyloid proteins, and an increase in fluorescence correlates with an increase in amyloid fibril formation, whereas a decrease in fluorescence correlates with a decrease in amyloid fibrils due to disassembly and/or disruption. The Alzheimer's Aß protein (1-42) when placed in solution, such as distilled water, tends to spontaneously form amyloid fibrils. Using this sensitive assay, any decreases or increases in fluorescence was previously shown to correlate with a decrease or increase in the amount of amyloid fibrils (H Naiki et al, Lab. Invest. 65:104-110, 1991; H Levine III, Protein Sci. 2:404-410, 1993; H Levine III, Amyloid: Int. J. Exp. Clin. Invest. 2:1-6, 1995; H Naiki and K. Nakakuki, Lab. Invest. 74:374-383, 1996), allowing one to identify, and quantitate the extent of potential inhibitors and/or enhancers of Alzheimer's Aß 1-42 amyloid fibrils.

To assess the effects of standardized green tea extract on potential disassembly/ disruption of preformed AB 1-42 fibrils, 50 µl of Aß 1-42 +/- standardized green tea leaf extract at various concentrations (described above) were added to 1.2ml of 100µM Thioflavin T (Sigma Chemical Co., St. Louis, MO) in 50mM NaPO4 (pH 6.0) for fluorometry readings (as described in Example 1).

As shown in Figure 3, increasing amounts of standardized green tea extract caused a dose-dependent disassembly/disruption of pre-formed Alzheimer's Aß 1-42 fibrils. Aß 1-42 alone demonstrated a mean fluorescence of 780 +/- 50 fluorescence units (Figure 3). Standardized green tea extract at 10µg/ml significantly (p<0.05) caused a disassembly/disruption of Aß 1-42 fibrils by 17+7%. On the other hand, 100µg/ml and 200µg/ml significantly (p<0.005) caused a disassembly/disruption of Aß 1-42 fibrils by 76+/-1.0% and 85+/-4.0%, respectively. This study demonstrated that standardized green tea extract and at least one of its catechin, bioflavanoid, flavanol, flavandiol, flavanoid or tannin active constituents caused disassembly/disruption of pre-formed Aß 1-42 amyloid fibrils and was effective in a dose-dependent manner.

### Example 3

### Disaggregation of Alzheimer's Disease Aß 1-42 Fibrils by Standardized Green Tea Leaf Extract

In the next study a Congo red-Aß spectrophotometric assay (Klunk et al, Anal. Biochem. 266:66-76, 1999) was modified to determine the effectiveness of standardized green tea leaf extract on disaggregation of Alzheimer's Aß 1-42 amyloid fibrils. For this assay, 25µM of Aß 1-42 (Bachem Inc., Torrance CA, Lot #516817) was incubated in triplicate for 4 days in distilled water at 37°C in the absence or presence of 400µg/ml of standardized green tea extract (obtained from two commercial sources) in Tris-buffered saline (TBS)(100 mM Tris; 50 mM NaCl; pH 7.0, with 0.02% sodium azide). The Aß: green tea extract weight ratio was 1:4. Source 1 of the standardized green tea extract used in this study was from Sundown Herbals (manufactured and distributed for Sundown Vitamins, Boca Raton, Fl), whereas source 2 of the standardized green tea extract used in this study was form Nature's Resource (Mission Hills, CA). The green tea used in this study were extracted in distilled water as described in Examples 1 and 2.

Following incubation of Aß 1-42 in the presence or absence of standardized green tea extracts (as described above)(standardized green tea extracts from the 2 sources are referred to as test compounds), 50µl of 360 µM of Congo red (Sigma Chemical Co. St. Louis, MO, USA) in distilled water was then added to 250 µl of each incubation mixture, giving a final AB:Congo red molar ratio of 1:3. After 10 minutes, the absorbance at 405 nm (reference wavelength to account for the absorbance of Congo red alone at 540nm) and 540 nm (sample absorbance where "sample" refers to Aß alone, test compound alone, or Aß + test compound, all in the presence of Congo red) was determined using a Biorad Model 550 ELISA Plate Reader (Biorad, Hercules, CA, USA). The absorbance at wavelength 405 nm was automatically subtracted by the ELISA plate reader from the absorbance at wavelength 540 nm (difference is referred to as Δ absorbance)(Klunk et al, Anal. Biochem. 266:66-76, 1999). Therefore, the Δ absorbance reading at 540nm was proportional to the amount of aggregated Aß left in solution (Klunk et al, Anal. Biochem. 266:66-76, 1999).

For all experiments involving test compounds, the Δ absorbance reading at 540nm of the test compound alone (in the absence of Aß), was always subtracted from the corresponding Δ absorbance reading at 540nm of the test compound in the presence of Aß.

Using this modification of the method of Klunk et al (Anal. Biochem. 266:66-76, 1999), the use of a greater final concentration of Congo red (i.e. 60µM instead of 14µM)(Anal. Biochem. 266:66-76, 1999), in the presence of fibrillar Aß gave an overall absorbance at 540 nm that was always below 1.0 Absorbance Unit (AU), and well within the linear absorbance range.

Standardized green tea leaf extracts from two commercial sources were tested using the above described Congo red-Aß spectrophotometric assay to determine their effectiveness on disaggregation of Aß 1-42 Alzheimer's amyloid fibrils.

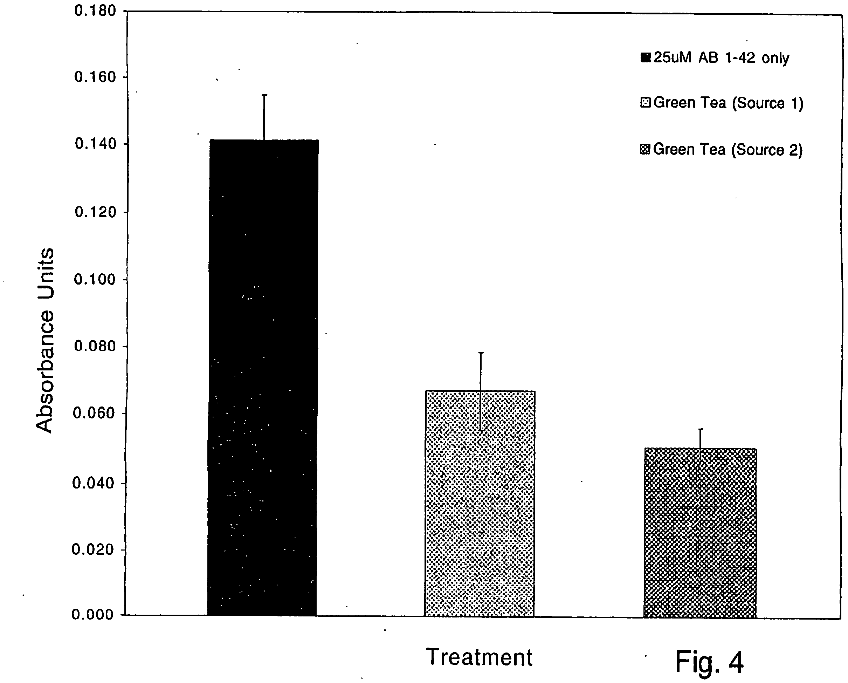

As shown in Figure 4, both commercially available standardized green tea extracts caused a disaggregation of pre-aggregated Aß 1-42 amyloid fibrils as determined using the Congo red spectrophotometric assay described above. Aß 1-40 alone demonstrated a mean Δ absorbance of 0.141+/- 0.013 AU (Figure 4). Standardized green tea extract from source 1 (Sundown Herbals) caused a significant (p<0.005) 52.5 +/- 8.5% disaggregation of 25 µM Aß 1-42 fibrils when incubated at a Aß 1-42:green tea extract weight ratio of 1:4 (Figure 4). On the other hand, standardized green tea extract from source 2 (Nature's Resource) caused a significant (p<0.005) 63.8 +/-4.2% disaggregation of 25 µM Aß 1-42 fibrils when incubated at a Aß 1-42:green tea extract weight ratio of 1:4 (Figure 4). Thus, independent of source, standardized green tea leaf extract and at least one of its catechin, bioflavanoid, flavanol, flavandiol, flavanoid or tannin active constituents was a potent disaggregator of Alzheimer's Aß 1-42 amyloid fibrils.

### Example 4

### Catechins are Potent Inhibitors of Alzheimer's Aβ (1-40) Amyloid Fibril Formation

A previously described method of measuring amyloid fibril formation utilizing Thioflavin T fluorometry (H Naiki et al, Lab. Invest. 65:104-110, 1991; H Levine III, Protein Sci. 2:404-410, 1993; H Levine III, Amyloid: Int. J. Exp. Clin. Invest. 2:1-6, 1995; H Naiki and K. Nakakuki, Lab._Invest. 74:374-383, 1996) was employed initially to identify whether standardized green tea leaf extract was capable of inhibiting Alzheimer's Aβ amyloid fibril formation.

In a first study, the effects of commercially available catechins as a potent Alzheimer's disease amyloid inhibitory agent on Alzheimer's Aβ (1-40) fibril formation was assessed by Thioflavin T fluorometry. The Alzheimer's Aβ protein (1-40) when incubated at 37°C tends to spontaneously form amyloid fibrils which increase in quantity over time. In this study, we tested for catechins to inhibit the Alzheimer's amyloid Aβ protein from forming fibrils over a 1 week period. For this study, 125 µM of Aβ (1-40)(Bachem Inc., Torrance, CA, USA) was incubated in microcentrifuge tubes at 37°C for 1 week (in triplicate), either alone, or in the presence of catechins from different commercial sources including Epicatechin (ICN Pharmaceuticals Inc., Costa Mesa, CA), Epicatechin (Aldrich, St. Louis, MO), Catechin hydrate (Fluka Chemica-Biochemika, Ronkonkoma, New York) and Catechin (Aldrich, St. Louis, MO) in 50 mM Tris HCl, 50 mM NaCl, pH 7.0 (TBS). Increasing amounts of catechins (as described above) were tested including Aβ:catechin at a 1:1, 1:0.1, 1:0.01 and 1:0.001 weight/weight ratio to determine if catechins exerted a dose-dependent inhibition of Aβ 1-40 fibril formation.

To assess the effects of catechins on Aβ (1-40) fibril formation, aliquots were taken from each tube for analysis at 0, 3 days, and 7 days. For each determination described above, following each incubation period, 10µl of Aβ +/- catechins were added to 1.2ml of 100µM Thioflavin T (Sigma Chemical Co., St. Louis, MO) in 50mM NaPO₄ (pH 6.0). Studies indicated that increasing concentrations of fibrillar Aβ gave a proportional increase in fluorescence in the presence of 100µM Thioflavin T, ruling out the presence of any disproportionate inner filter effects in these studies. Fluorescence emission at 482 nm was measured on a Turner instrument-model 450 fluorometer at an excitation wavelength of 450 nm. For each determination, the fluorometer was calibrated by zeroing in the presence of the Thioflavin T reagent alone, and by setting the 50 ng/ml riboflavin (Sigma Chemical Co., St. Louis, Mo) in the Thioflavin T reagent to 1800 fluorescence units. All fluorescence determinations were based on these references and any fluorescence given off by any of the compounds in the presence of the Thioflavin T reagent was always subtracted from all readings.

For all fibrillogenesis studies utilizing Thioflavin T fluorometry, as disclosed herein, comparisons of amyloid protein in the presence or absence of catechins were based on paired Student's t tests with data shown as mean +/- standard deviation. Significance was reported at the 95% (p< 0.05), 99% (p<0.01) and 99.5% (p<0.005) confidence levels.

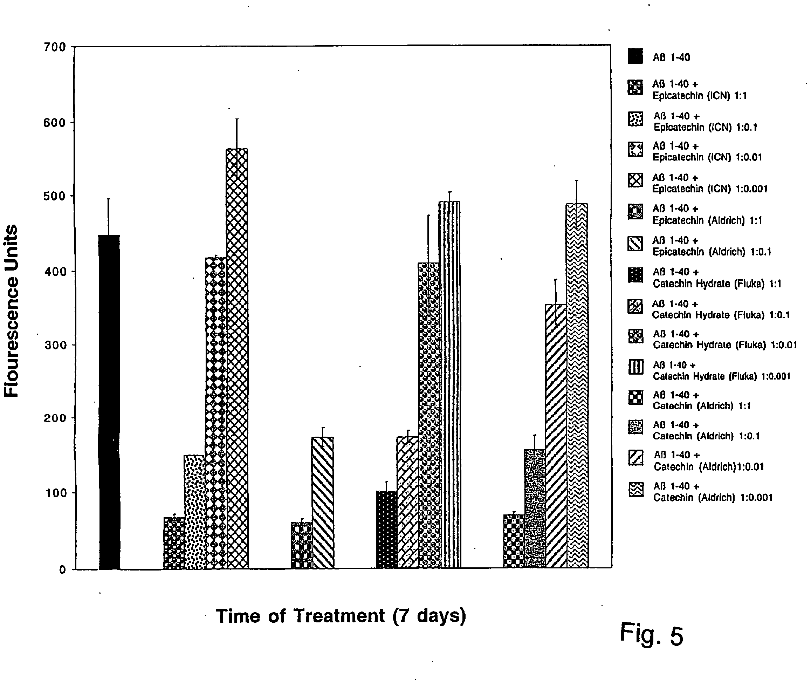

The effects of commercially available catechins on Alzheimer's Aβ (1-40) amyloid fibril formation was evaluated over a 1-week incubation period. During the 1-week incubation period, there was a gradual increase in the fluorescence of Aβ (1-40) alone, with a peak fluorescence of 450+/- 46 fluorescence units observed at 7 days (Figure 5), consistent with previous studies (Castillo et al, J. Neurochem. 69:2452-2465, 1997). Epicatechin (from ICN and Aldrich), catechin hydrate, and catechin all inhibited Aβ (1-40) amyloid fibril formation in a dose-dependent manner, and significantly (p<0.01) at Aβ:catechin weight ratios of 1:1 and 1:0.1 (Figure 5). A 1:1 Aβ:epicatechin (ICN) weight/weight ratio inhibited Aβ fibril formation by 85%, whereas a 1:0.1 Aβ:epicatechin (ICN) weight/weight ratio inhibited Aβ fibril formation by 67%. A 1:1 Aβ:epicatechin (Aldrich) weight/weight ratio inhibited Aβ fibril formation by 86%, whereas a 1:0.1 Aβ:epicatechin (ICN) weight/weight ratio inhibited Aβ fibril formation by 62%. A 1: 1 Aβ:catechin hydrate (Aldrich) weight/weight ratio inhibited Aβ fibril formation by 88%, whereas a 1:0.1 Aβ:catechin (Aldrich) weight/weight ratio inhibited Aβ fibril formation by 62%. A 1:1 Aβ:catechin (Aldrich) weight/weight ratio inhibited Aβ fibril formation by 85%, whereas a 1:0.1 Aβ:catechin (Aldrich) weight/weight ratio inhibited Aβ fibril formation by 66%. This initial data indicated that catechins, including epicatechin, catechin hydrate and catechin were potent inhibitors of Alzheimer's amyloid fibril formation.

### Example 5

### Inhibition of Aβ Amyloid Fibril Formation by Catechins as Demonstrated by a Reduction in Congo red Birefringence

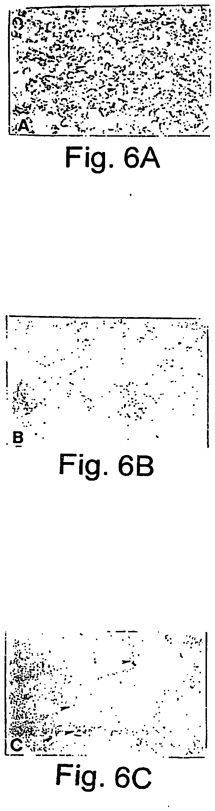

The inhibition of Aβ 1-40 fibril formation by catechins was confirmed by a loss of Congo red birefringence as demonstrated in Congo red staining assays (Figure 6). In these studies, aliquots of 125 µM Aβ only, or Aβ + catechins were incubated for 1 week at 37°C, and then placed on gelatin-coated slides, air-dried overnight and stained with Congo red. A marked reduction in Congo red birefringence (as viewed under polarized light) was exhibited in the presence of epicatechin (ICN)(as an example)(Figure 6) at an Aβ:epicatechin weight ratio of 1:0.5 (Fig. 6B) and to a lesser extent at an Aβ:epicatechin weight ratio of 1:0.05 (Fig. 6C). Figure 6A demonstrates the Congo red staining of Aβ amyloid deposits following incubation of 125 µM of Aβ 1-40 at 37°C for 1 week alone. This study demonstrated that catechins, such as epicatechin, was a potent inhibitor of Aβ amyloid fibril formation.

### Example 6

### Disassembly/Disruption of Alzheimer's Disease Aβ 1-42 Amyloid Fibrils by Catechins

In the next study, commercially available catechins were tested for their ability to cause a disassembly/disruption of pre-formed Alzheimer's disease amyloid fibrils containing Aβ 1-42. This type of activity would be important for any potential anti-amyloid compound that can be used in patients who already have substantial amyloid deposition in organs and/or tissues. For example, Alzheimer's disease patients in mid-to-late stage disease have abundant Aβ-containing amyloid deposits in their brains as part of both neuritic plaques and cerebrovascular amyloid deposits. A compound capable of causing disassembly/disruption of pre-existing amyloid deposits would be advantageous for use in these patients who are at latter stages of the disease process.

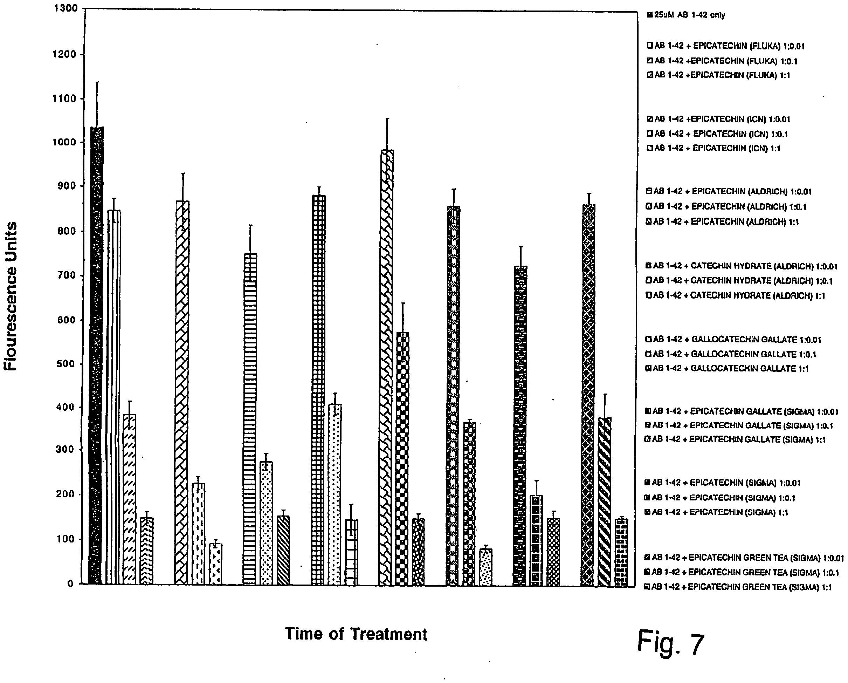

For this study, 1 mg of Aβ 1-42 (Bachem Inc., Torrance, CA, USA) was dissolved in 1.0 ml of double distilled water (1 mg/ml solution). 25 µM of Aβ 1-42 was then incubated at 37°C, in the absence or presence of epicatechin (from Fluka), epicatechin (from ICN), catechin hydrate (from Aldrich), Gallocatechin gallate (from Sigma), Epicatechin gallate (from Sigma), Epicatechin (from Sigma), Epicatechin green tea (from Sigma) in the presence of 150 mM Tris HCl, 10 mM NaCl (pH 7.0) with 0.02% sodium azide. In these studies (see results described below and in Figure 7), the Aβ 1-42:catechin weight ratios tested 1:1, 1:0.1 and 1:0.01. The Thioflavin T fluorometry assay as described in Example 1 was used.

As shown in Figure 7, increasing amounts of various commercially available catechins caused a dose-dependent disassembly/disruption of pre-formed Alzheimer's Aβ 1-42 fibrils. Aβ 1-42 alone demonstrated a mean fluorescence of 1037 +/- 101 fluorescence units (Figure 7). Epicatechin (from Fluka, ICN, Aldrich and Sigma), catechin hydrate, gallocatechin gallate, and epicatechin gallate all caused a disruption/disassembly of pre-formed Aβ (1-42) amyloid fibrils in a dose-dependent manner, and significantly (p<0.01) at Aβ:catechin weight ratios of 1:1 and 1:0.1 (Figure 6). A 1:1 Aβ:epicatechin (Fluka) weight/weight ratio disrupted Aβ 1-42 fibril formation by 86%, whereas a 1:0.1 Aβ:epicatechin (Fluka) weight/weight disrupted Aβ 1-42 fibril formation by 63%. A 1:1 Aβ:epicatechin (ICN) weight/weight ratio disrupted Aβ 1-42 fibril formation by 91%, whereas a 1:0.1 Aβ:epicatechin (ICN) weight/weight disrupted Aβ 1-42 fibril formation by 78%. A 1:1 Aβ:epicatechin (Aldrich) weight/weight ratio disrupted Aβ 1-42 fibril formation by 85%, whereas a 1:0.1 Aβ:epicatechin (Aldrich) weight/weight disrupted Aβ 1-42 fibril formation by 74%. A 1:1 Aβ:catechin hydrate (Aldrich) weight/weight ratio disrupted Aβ 1-42 fibril formation by 86%, whereas a 1:0.1 Aβ: catechin hydrate (Aldrich) weight/weight disrupted Aβ 1-42 fibril formation by 60%. A 1:1 Aβ:gallocatechin gallate (Sigma) weight/weight ratio disrupted Aβ 1-42 fibril formation by 86%, whereas a 1:0.1 Aβ: gallocatechin gallate (Sigma) weight/weight disrupted Aβ 1-42 fibril formation by 44%. A 1:1 Aβ:epicatechin gallate (Sigma) weight/weight ratio disrupted Aβ 1-42 fibril formation by 92%, whereas a 1:0.1 Aβ: epicatechin gallate (Sigma) weight/weight disruptedAβ 1-42 fibril formation by 65%. A1:1 Aβ:epicatechin (Sigma) weight/weight ratio disrupted Aβ 1-42 fibril formation by 85%, whereas a 1:0.1 Aβ: epicatechin (Sigma) weight/weight disruptedAβ 1-42 fibril formation by 81%. A 1:1Aβ:epicatechin green tea (Sigma) weight/weight ratio disrupted Aβ 1-42 fibril formation by 86%, whereas a 1:0.1 Aβ: epicatechin green tea (Sigma) weight/weight disrupted Aβ 1-42 fibril formation by 63%. This study demonstrated that the catechins, including epicatechin (from Fluka, ICN, Aldrich and Sigma), catechin hydrate (Aldrich), gallocatechin gallate (Sigma), and epicatechin gallate all caused a disassembly/disruption of pre-formed Aβ 1-42 amyloid fibrils and was effective in a dose-dependent manner.

### Example 7

### Catechins are Potent Inhibitors of Parkinson's Fibril Formation

In the next study, commercially available catechins were tested for their ability to inhibit Parkinson's alpha-synuclein fibril formation. NAC (also known as, and sometimes referred to herein as, NAC-P), a 35 amino acid fragment of alpha-synuclein was used since it is know to spontaneously form amyloid-like fibrils following incubation at 37°C. For this study, 62.5 µM of NAC (Bachem Inc., Torrance, CA, USA) was incubated in microcentrifuge tubes at 37°C for 1 week (in triplicate), either alone, or in the presence of catechins from different commercial sources including Epicatechin (EC)(Fluka), Epicatechin (EC)(ICN), Epicatechin (EC)(Aldrich), Epicatechin (EC)(Sigma), Epicatechin gallate (Sigma), Catechin hydrate (Aldrich), and Gallocatechin gallate (Sigma) in 150 mM Tris HCl, 10 mM NaCl, pH 7.0 (TBS). Increasing amounts of catechins (as described above) were tested including NAC:catechin at a 1:1, 1:0.5, 1:0.1, and 1:0.01 weight/weight ratio to determine if catechins exerted a dose-dependent inhibition of NAC fibril formation. To assess the effects of catechins on NAC fibril formation, aliquots were taken from each tube for analysis at 0, 3 days, and 7 days using the Thioflavin T fluorometry procedure as described in Example 4.

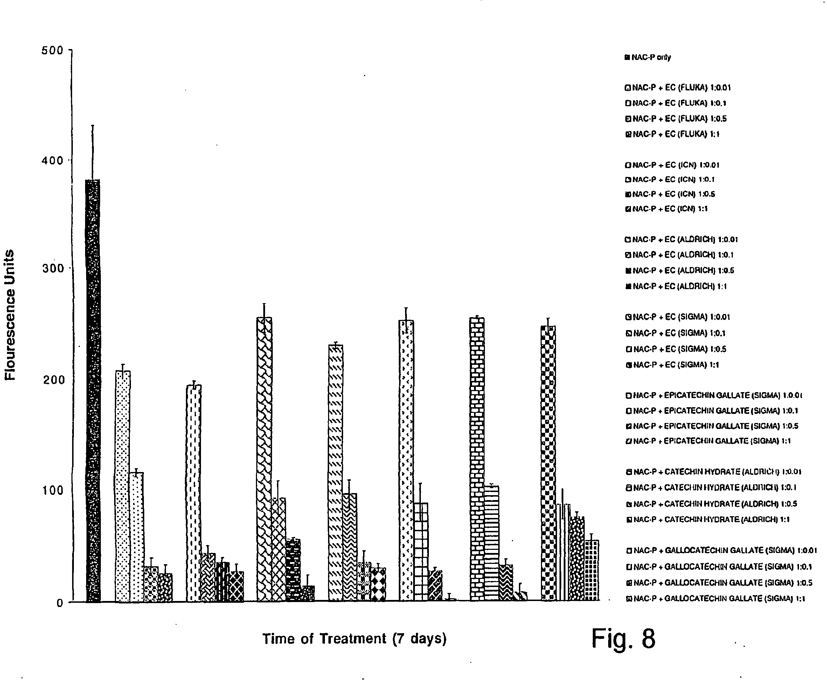

The effects of commercially available catechins on NAC fibril formation was evaluated over a 1-week incubation period. During the 1-week incubation period, there was a gradual increase in the fluorescence of NAC, with a peak fluorescence of 381 +/-50 fluorescence units observed at 7 days (Figure 8). Epicatechin (from Fluka, ICN, Aldrich and Sigma), epicatechin gallate (Sigma), catechin hydrate (Aldrich), and gallocatechin gallate (Sigma) all inhibited NAC fibril formation in a dose-dependent manner, and significantly (p<0.01) at NAC:catechin weight ratios of 1:1, 1:0.5, 1:0.1 and 1:0.01 (Figure 8). A 1:0.1 NAC:epicatechin (Fluka) weight/weight ratio inhibited NAC fibril formation by 70%, whereas a 1:0.01 NAC:epicatechin (Fluka) weight/weight ratio inhibited NAC fibril formation by 46%. A 1:0.1 NAC:epicatechin (ICN) weight/weight ratio inhibited NAC fibril formation by 89%, whereas a 1:0.01 NAC:epicatechin (ICN) weight/weight ratio inhibited NAC fibril formation by 49%. A 1:0.1 NAC:epicatechin (Aldrich) weight/weight ratio inhibited NAC fibril formation by 76%, whereas a 1:0.01 NAC:epicatechin (Aldrich) weight/weight ratio inhibited NAC fibril formation by 33%. A 1:0.1 NAC:epicatechin (Sigma) weight/weight ratio inhibited NAC fibril formation by 75%, whereas a 1:0.01 NAC:epicatechin (Sigma) weight/weight ratio inhibited NAC fibril formation by 40%. A 1:0.1 NAC:epicatechin gallate (Sigma) weight/weight ratio inhibited NAC fibril formation by 77%, whereas a 1:0.01 NAC:epicatechin gallate (Sigma) weight/weight ratio inhibited NAC fibril formation by 34%. A 1:0.1 NAC:catechin hydrate (Aldrich) weight/weight ratio inhibited NAC fibril formation by 77%, whereas a 1:0.01 NAC:catechin hydrate (Aldrich) weight/weight ratio inhibited NAC fibril formation by 34%. A 1:0.1 NAC:gallocatechin gallate (Sigma) weight/weight ratio inhibited NAC fibril formation by 73%, whereas a 1:0.01 NAC:gallocatechin gallate (Sigma) weight/weight ratio inhibited NAC fibril formation by 34%. This study demonstrated that the catechins, including epicatechin (from Fluka, ICN, Aldrich and Sigma), catechin hydrate (Aldrich), gallocatechin gallate (Sigma), and epicatechin gallate (Sigma) all caused an inhibition of NAC fibril formation and was effective in a dose-dependent manner.

### Example 8

### Screening and Identifying Specific Polyhydroxylated Aromatic Compounds as Inhibitors of AA Amyloidosis Using a Cell Culture Model System

It is believed that specific polyhydroxylated aromatic compounds are potent inhibitors/disrupters of fibrillar AA amyloid formation and deposition in cell culture. A group of about 30 commercially available polyhydroxylated aromatic compounds that differ by position of various functional hydroxyl groups are believed to inhibit fibrillar AA amyloid formation and deposition using a cell culture model system. A murine macrophage cell culture system receiving recombinant serum amyloid A 2 (SAA2) and amyloid enhancing factor (AEF) is used to generate fibrillar AA amyloid *in vitro.* Fibrillar AA amyloid deposition detected by Congo red staining and Thioflavin S fluorescence is quantitated using an image analysis system. In addition, quantitation of AA amyloid formation and deposition is achieved by the analysis of the cell layer and media derived from cultures using SDS-PAGE, immunoblotting and scanning densitometry. Selected efficacious compounds found to inhibit AA amyloid formation and/or deposition in culture are then tested in an experimental mouse model of AA amyloidosis.

### Example 9

### Determining the Efficacy of Selected Polyhydroxylated Aromatic Compounds in a Mouse Model of Systemic AA Amyloidosis

It is believed that specific polyhydroxylated aromatic compounds are potent inhibitors of fibrillar AA amyloid formation and deposition in a mouse model of experimental AA amyloidosis. Selected efficacious compounds identified as above are used to orally treat (by gavage) groups of CBA/J mice (n=8 per group) over a 21-day treatment period. Fibrillar AA amyloid is induced in these animals on day 8 of the treatment period by a single subcutaneous injection of silver nitrate (inflammatory stimulus), followed by a single tail-vein injection of 100µg AEF. A group of 12 animals orally treated with phosphate-buffered saline (PBS) and induced for AA amyloidosis (on day 8) serve as the control group. Spleen, liver and kidneys from each animal are obtained and the % amyloid burden in each organ are quantified using image analysis following Congo red staining or Thioflavin S fluorescence. In addition, immunohistochemistry is implemented for the detection ofAA amyloid protein and its precursor (i.e. SAA), as well as other known AA amyloid-associated components (i.e. heparan sulfate proteoglycans/glycosaminoglycans, amyloid P component, and ApoE) to determine if reduction of AA amyloid deposition in tissues also correlates with reduction in amyloid-associated components.

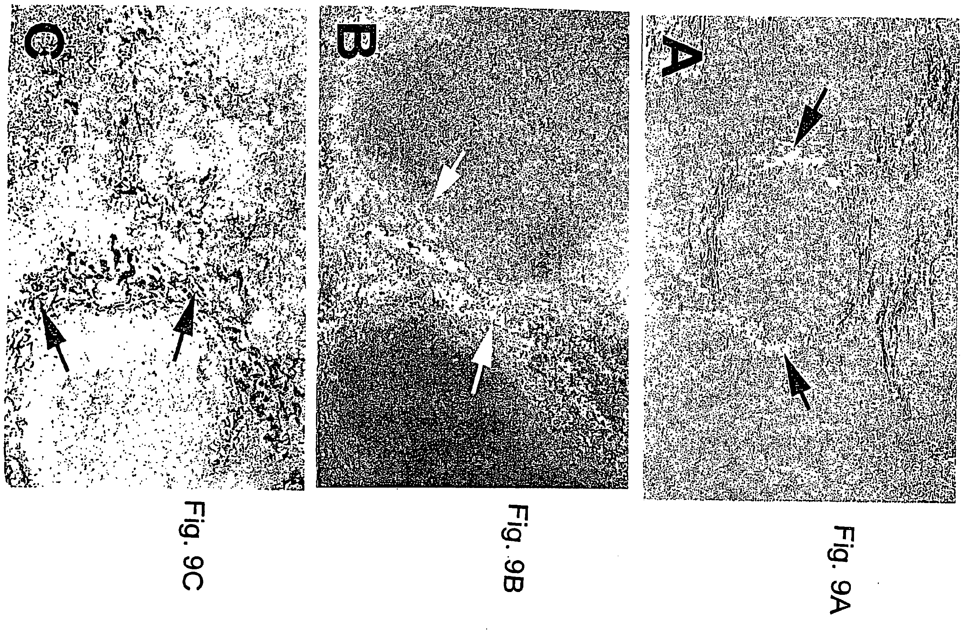

We have established the use of the experimental AA mouse model in-house. AEF was first kindly provided by Dr. Robert Kisilevsky (Queen's University, Kingston, Ontario, Canada). CBA/J female mice were then injected subcutaneously with a single 0.5ml injection of 3% silver nitrate (to induce an inflammatory reaction) and a single tail-vein injection of 100µg AEF. Using this model within 2-3 days following injection, abundant AA amyloid deposition occurred in spleen, followed by liver and kidney. As shown in Figure 9, fibrillar AA amyloid deposits were first observed in the perifollicular areas of the spleen. Congo red staining of tissue sections revealed a characteristic red/green birefringence (as viewed under polarized light) indicative of fibrillar amyloid deposits (Fig. 9A). The presence of fibrillar amyloid deposits was also confirmed by positive Thioflavin S fluorescence (Fig.9C).

Confirmation of AA amyloid deposits in spleen, liver and kidney was demonstrated by positive immunostaining using a polyclonal antibody against the AA amyloid protein (Serotek)(not shown). In addition, as previously described in a number of studies (Snow et al, Lab. Invest. 53;37-44, 1985; Snow et al, Lab. Invest. 56:665-675, 1987; Snow et al, Lab. Invest. 57:687-698, 1987; Snow et al, J. Histochem. Cytochem. 39:1321-1330, 1991.) AA amyloid deposits demonstrated a co-localization of heparan sulfate glycosaminoglycans (likely as part of proteoglycans) as shown using a phage display generated periplasmic fraction antibody (known as HS4C3 which recognizes a specific heparan sulfate GAG epitope)(van Kuppevelt et al, J. Biol. Chem. 273:12960-12966,1998.)(Fig. 9C).

Figure 9 shows amyloid deposition in spleen in a mouse model of experimental AA amyloidosis. Panels represent splenic tissue taken 5 days after CBA/J mice were injected with 100µg AEF + 0.5ml of 3% silver nitrate. (Fig. 9A) Splenic amyloid in the perifollicular area (bright areas; arrows) as shown by Congo red staining (i.e. red/green birefringence) as viewed under polarized light. (Fig. 9B) Splenic amyloid (bright areas; arrows) in the perifollicular area in another animal as demonstrated by positive Thioflavin S fluorescence. (Fig. 9C) Co-localization of heparan sulfate glycosaminoglycans in the splenic perifollicular areas (dark areas; arrows) as demonstrated by immunostaining with a phage-display generated antibody (known as HS4C3), that recognizes a specific heparan sulfate GAG epitope. All figures X100.

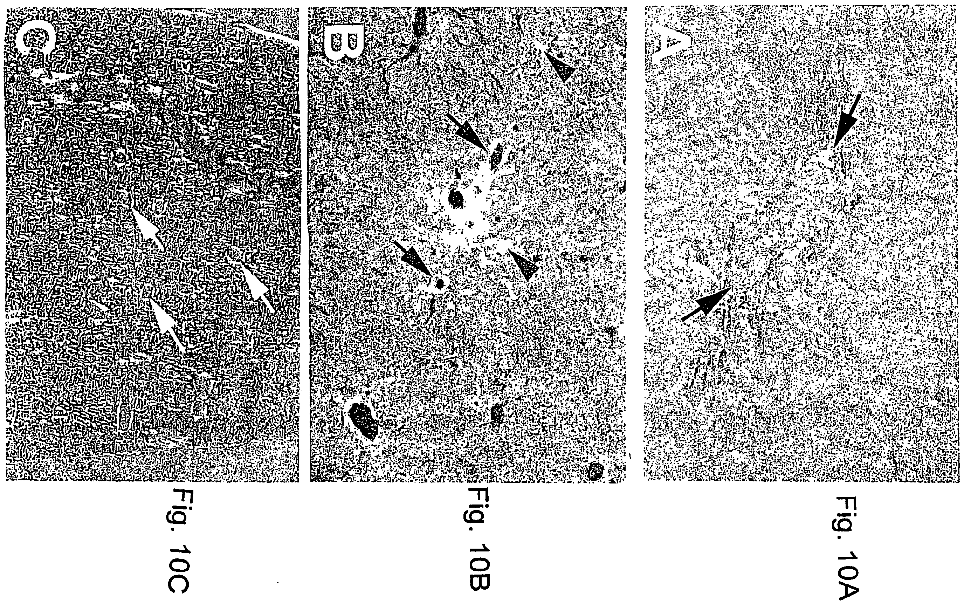

In the liver, fibrillar AA amyloid deposits were first observed within 3-4 days in the walls of the central veins (Fig. 10A), followed by amyloid deposition throughout the parenchyma by days 5-7 (Fig. 10C). The nature of fibrillar amyloid deposits was confirmed by both positive staining with Congo red (i.e. red/green birefringence as viewed under polarized light)(Figs. 10A, 10C) and Thioflavin S (i.e. positive fluorescence)(Fig. 10B).

Figure 10 shows amyloid deposition in liver in a mouse model of experimental AA amyloidosis. Panels A and B represent liver tissue taken 7-10 days after CBA/J mice were injected with 100µg AEF + 0.5ml of 3% silver nitrate. (Fig. 10A) Liver amyloid (at 7 days) primarily confined to the walls of a central vein (bright areas; arrows) as shown by Congo red staining (i.e. red/green birefringence) as viewed under polarized light (arrows). X100 (Fig. 10B) Liver amyloid (at 7 days) in the walls of central veins (bright areas; arrows) and penetrating into the parenchyma (arrowheads) as detected by Thioflavin S fluorescence. X100 (Fig. 10C) A lower magnification photomicrograph demonstrating marked amyloid accumulation (at 10 days) in the walls of central veins (bright areas; arrowheads) and throughout the parenchyma in liver following 10 days after injection of AEF + AgNO₃. X25.

These studies indicate that the experimental mouse model of systemic AA amyloidosis is viable. We utilize this model to test the efficacy of AA amyloid inhibitors following initial screening using the cell culture model system.

### Example 10

### Identification of a New Class of Compounds as Potential Anti-Amyloid Agents

Most of our previous studies involved the analysis of these compounds for the disruption/disassembly of pre-formed amyloid fibrils consisting of beta-amyloid protein (observed in Alzheimer's disease amyloid deposits), islet amyloid polypeptide (observed in the islets of 90% of patients with type 2 diabetes), alpha-synuclein (observed in Parkinson's disease deposits), or prion protein (observed in prion diseases). Our initial studies demonstrate that specific polyphenolic compounds can cause disruption/disassembly of different types of amyloid proteins, and thus tend to be less specific, whereas other polyphenolic compounds tend to only disrupt one type of amyloid protein (and are thus more specific). Examples of some of the polyhydroxylated aromatic compounds which we have screened for inhibition of AA amyloid formation and deposition are set out below.

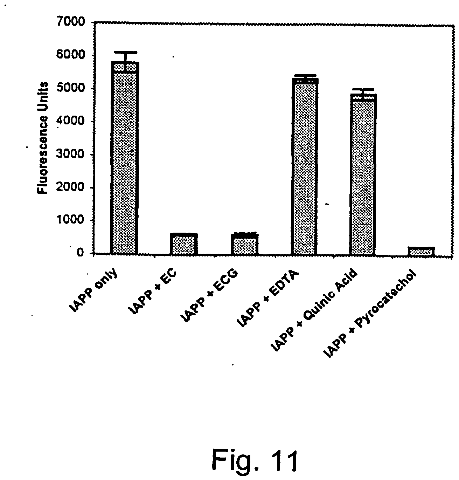

As an example, Figure 11 demonstrates the effect of specific polyhydroxylated aromatic compounds to disrupt/disassemble amyloid fibrils consisting of islet amyloid polypeptide (IAPP)(the amyloid protein that accumulates in the pancreatic islets of ∼90% of patients with type 2 diabetes)(Westermark, 1972; Clark et al, 1988) as assessed by Thioflavin T fluorometry (Naiki et al,1991; Levine III,1993;1995). In this assay, increases or decreases in Thioflavin T fluorometry is proportional to increases or decreases in the quantity of IAPP fibrils (Naki et al, 1991; Levin III, 1993; 1995). As shown in Figure 11, following a 1-week incubation at 37°C, 25 µM of IAPP alone demonstrated 5,806 +/- 301 fluorescence units indicating the presence of abundant IAPP amyloid fibrils. IAPP fibrils incubated for 1 week at 37°C at an IAPP: compound weight ratio of 1:0.1 (approximate molar ratio of 1:1) with specific hydroxylated aromatic compounds including epicatechin (EC; Fluka), epicatechin gallate (ECG; Sigma) or pyrocatechol (Sigma) all demonstrated a marked 90-96% disruption/disassembly of pre-formed IAPP fibrils. On the other hand, EDTA (as a negative control) and the polyphenolic known as quinic acid did not cause any significant disruption/disassembly of pre-formed IAPP fibrils (Fig. 11). The direct disruption of pre-formed IAPP fibrils by specific polyphenolic compounds was also confirmed by Congo red staining assays (not shown).

Figure 11 shows specific polyhydroxylated aromatic compounds act as potent disruptors of pre-formed amyloid fibrils. This graph is a 1-week Thioflavin T fluorometry assay to identify inhibitors of IAPP fibrils. As shown, only epicatechin (EC), epicatechin gallate (ECG) and pyrocatechol disrupt/disassemble pre-formed IAPP fibrils by 90-96%.

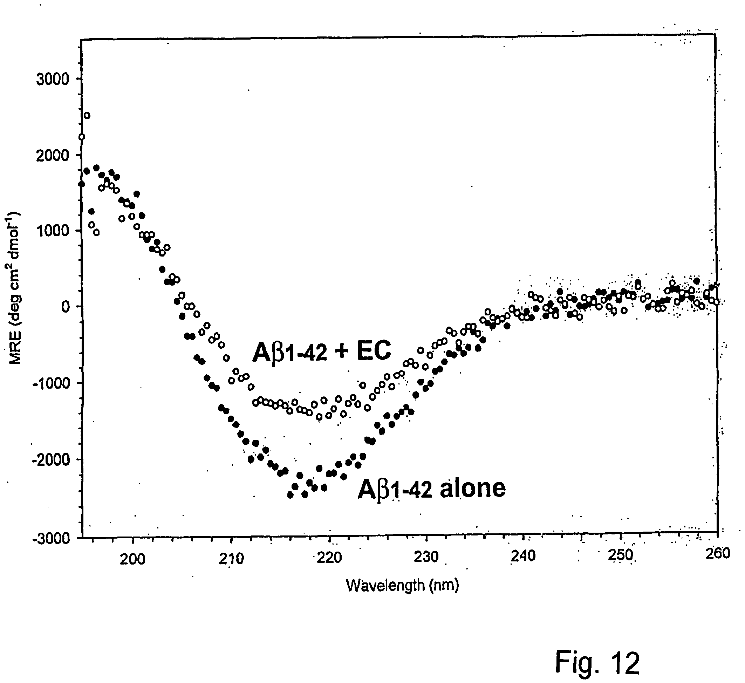

In our other studies, circular dichroism spectroscopy is utilized to test the efficacy of certain polyphenolics to disrupt pre-formed fibrils consisting of beta-amyloid protein (Aß) 1-42, a protein that forms amyloid fibrils as part of extracellular amyloid plaques in the brains of all patients with Alzheimer's disease. As an example, the effects of the polyphenolic, epicatechin on the disruption of Aß 1-42 fibrils was tested. In this study, 50µM of Aß 1-42 (Bachem Inc) was incubated in the absence or presence of epicatechin (EC)(Sigma) for 1 week at 37°C, at an Aß:EC wt/wt ratio of 1:1 (approximate molar ratio of 1:10). As shown in Figure 12, Aß 1-42 alone demonstrates the typical spectra of a fibrillar amyloid protein containing ß-pleated sheet structure by the marked minima observed at 222 nm. On the other hand, incubation with epicatechin by 3 days caused a significant disruption/disassembly of ß-pleated sheet structure (Fig. 12), suggesting that specific polyhydroxylated aromatic compound's mechanism of action may involve ß-pleated sheet disruption.

Figure 12 shows disruption/disassembly of ß-pleated sheet structure of pre-formed Alzheimer's disease amyloid fibrils by epicatechin. Circular dichroism spectroscopy was used to assess the ability of epicatechin (EC) to cause a disruption/disassembly of ß-pleated sheet structure of pre-formed Aß 1-42 fibrils. As shown, by 3 days of co-incubation epicatechin was effective in disrupting the ß-sheet structure (as revealed by the change in the spectra, especially at 222 nm).

### Example 11

### Inhibition of Experimental AA Amyloid Amyloidosis by Epicatechin

Studies also demonstrate that the specific polyhydroxylated aromatic compound, known as epicatechin (C₁₅H₁₄O₆; MW 290.3) has the ability to markedly prevent AA amyloid deposition in spleen and liver in the mouse model (i.e. following AEF + silver nitrate administration). This was not observed following treatment with catechin (the epimer of epicatechin), suggesting that structure-activity relationships exist for the inhibition of AA amyloid fibril formation and deposition by polyhydroxylated aromatic compounds.

In this study, 4 groups (n= 6 per group) of female CBA/J 8-week old mice were first pre-treated for 7 days with either a) phosphate-buffered saline (PBS), b) epicatechin (C₁₅H₁₄O₆; MW 290.3)(Fluka, St. Louis, MO) or c) catechin hydrate (C₁₅H₁₄O₆; MW 290.3)(Fluka, St. Louis, MO). Epicatechin was administered daily by oral gavage (0.5 ml of test article in PBS, pH 7.4) at a dosage of 78 mg/kg/day (solubility limit of epicatechin), whereas catechin hydrate was administered at a high dose (500 mg/kg/day).

Following a 7-day pretreatment period with either PBS, epicatechin or catechin, all animals were induced for AA amyloid deposition by a single subcutaneous injection of 0.5 ml of silver nitrate (inflammatory stimulus), followed by a single tail vein injection of 100µg of AEF in distilled water. The mice were then treated for another 14 days with PBS, epicatechin or catechin by oral gavage as described above (total test compound treatment time of 21 days). At the end of the experimental protocol, the animals were sacrificed and the spleen, liver and kidneys were taken for histological analysis.

Tissue sections (i.e. spleen, liver and kidney) were then stained for fibrillar amyloid using both Congo red and Thioflavin S. Five sections per organ was then used to evaluate amyloid load. In these studies, an arbitrary scoring system was used (from 0 to 5) and amyloid load per organ was determined by the blind scoring of two investigators. As an example, for Congo red scoring of tissue sections in spleen, 0 = no Congo red birefringence in tissue; 1= slight Congo red birefringence confined to one portion of the perifollicular area; 2= Congo red birefringence in confined to two or more portions of the prelifollicular area; 3 = Congo red birefringence occupying the entire perifollicular area; 4= Congo red birefringence occupying the entire perifollicular area and penetrating into the white pulp; 5=Congo red birefringence occupying the entire perifollicular area and penetrating deep into white pulp. We also use a more quantitative approach to determine % amyloid load in various tissues using a more sophisticated image analysis system.

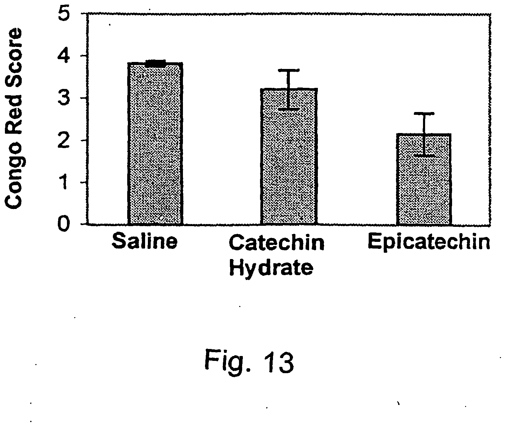

As shown in Figure 13, animals given saline alone demonstrated marked amyloid deposition in spleen by the end of the 2-week amyloid induction period, with a Congo red score of 3.83 +/- 0.1. Catechin hydrate (i.e. Congo red score of 3.20 +/- 0.46) reduced AA amyloid deposition in spleen by 16.5%, but this was not significant. However, epicatechin (i.e. Congo red score of 2.16 +/- 0.50) significantly (p<0.01) inhibited fibrillar AA amyloid deposition in spleen by 43.7% (Fig. 13). A similar inhibition of fibrillar AA amyloid in liver and kidney was also found following treatment with epicatechin, but not catechin (not shown).

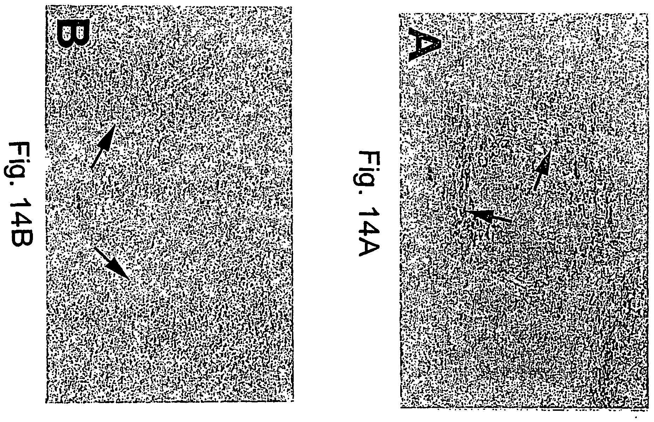

The inhibitory effect by epicatechin is so potent, that a few of the animals even demonstrated little to no fibrillar AA amyloid in tissues following treatment (Figure 14). This study demonstrates that selected polyhydroxylated aromatic compounds have the ability to serve as potential therapeutics for the treatment of systemic AA amyloidosis. The studies described use cell culture techniques and an experimental mouse model of AA amyloidosis to identify polyhydroxylated aromatic compounds that serve as lead compounds for the treatment of AA amyloidosis.

Figure 14 shows a marked reduction in splenic AA amyloid deposition by epicatechin as revealed by Congo red staining. (Fig. 14A) Congo red staining of AA amyloid deposits in the perifollicular area of the spleen (arrows) in a mouse induced with AEF + AgNO₃ (on day 8), and treated with PBS (for 21 days). Color photomicrographs demonstrate a red/green birefringence indicative of fibrillar amyloid (as viewed under polarized light). (Fig. 14B) Near complete elimination ofAA amyloid deposits in the perifollicular area of the spleen (arrows) as shown by lack of Congo red staining (as viewed under polarized light) of an animal induced with AEF + AgNO₃ (day 8), and treated with epicatechin (for 21 days).

### Example 12

### Identification of New AA Amyloid Inhibitors - In Vitro Screening

Our studies suggests that certain polyhydroxylated aromatic compounds, such as epicatechin, may serve as a potent inhibitor of fibrillar AA amyloid formation and deposition in tissues. We use innovative cell culture techniques to screen a number of known and commercially available polyhydroxylated aromatic compounds which differ by the number of aromatic rings and/or by the position of various hydroxyl and other function groups on the aromatic rings. These structure-activity relationship (SAR) type of screening studies give insight into the important structural characteristics needed for effective inhibition of AA amyloid formation and deposition. A murine macrophage culture system utilizing recombinant SAA2 and AEF is used to generate fibrillar AA amyloid *in vitro*. Fibrillar AA amyloid deposition detected by Congo red staining and Thioflavin S fluorescence is quantitated in culture using an image analysis system equipped for fluorescence and polarization. In addition, quantitation of AA amyloid formation is achieved by the analysis of the cell layer and media using SDS-PAGE, immunoblotting and scanning densitometry. When AA amyloid is established in culture, cultures are screened with selected polyhydroxylated compounds at increasing concentrations. The most efficacious compounds found to inhibit AA amyloid formation and deposition in culture are then tested in a relevant experimental mouse model of AA amyloidosis.

### Methodology:

### Establishment of a Cell Culture System for the Study of AA Amyloidosis

For the establishment of a cell culture system in which to screen for inhibitors of fibrillar AA amyloid formation and deposition, we use the method as described by Kluve-Beckerman et al (1999).

### Collection and Culture of Murine Macrophape/Peritoneal Cells:

Cells are collected by lavage from the peritoneal cavity of normal 8-10 week old female C57B1/6 mice. Briefly, 8 ml of collection medium (RPMI 1640; Life Technologies, Grand Island, NY), 25 mM HEPES, pH 7.0 and 1X antibiotic-antimycotic (Life Technologies) is injected intraperitoneally, the abdomen is gently massaged, and the fluid and cells are withdrawn. Cells are collected by centrifugation, resuspended at a concentration of 5X10⁶ cells/ml, and plated at a density of 1. 7 X 10⁶ cells/well in 8-well chamber slides. Culture media contain RPMI 1640, 2 mM L-glutamine, 1X antibiotic-antimycotic, and 15% fetal calf serum (Hyclone Labs, Logan, VT). Cells are allowed to attached for 3 hours, after which wells are rinsed thoroughly to remove nonadherent cells. Adherent cells display a uniform, round morphology typical of freshly plated, nonactivated macrophages. Cells are maintained in 350µl of culture medium per well at 37°C in an atmosphere of 5% CO₂, with media being replaced every 2-3 days. Following the addition of AEF and recombinant SAA2 (as described below) for the induction of AA amyloid deposition in culture (usually observed within 2-4 days of induction), cell cultures are maintained for a period of 14-20 days.

### Preparation of Amyloid Enhancing Factor:

AEF is prepared from the spleens of amyloidotic mice following a 2-week treatment with a single subcutaneous 0.5ml injection of AgNO₃, and a single tail vein injection of 100µg AEF in distilled water. The initialAEF material was kindly supplied by Dr. Robert Kisilevsky (Queen's University, Canada) to which more AEF was generated using the method of Pras et al (1968). Briefly, approximately 2 grams (10 amyloidotic spleens) of tissue is processed for each preparation. The spleens are homogenized using a Kontes tissue grinder in 40mls of 0.15M NaCl saline solution, until the tissue is in a fine slurry. The slurry is then centrifuged on a table-top microcentrifuge at 10,000 rpm for 30 minutes. The supernatant is discarded and the pellet is resuspended in 40mls of saline and centrifuged again as described above. This process is repeated seven times. The salt is then be removed from the pellets by rehomogenizing in sterile distilled water, and centrifugation on a table-top microcentrifuge at 15,000 rpm for 2 hours. The supernatant is then discarded and the pellet is resuspended in 20mls of distilled water, and then recentrifuged at 15, 000 rpm for 2 hours. This time the supernatant is not discarded and is designated as Supernatant II (Sup II), which contains significant AEF activity. The process used for the generation of Sup II is repeated, and the supernatant, designated as Sup III is saved. The pellet obtained from the last step is resuspended in 7 mls of distilled water and centrifuged at 10,000 rpm for 3 hours. The supernatant is saved and designated as Sup IV. Sup II, Sup III, and Sup IV contain AEF activity which is assayed by the Bradford method (Bradford, 1976) to determine the protein concentration for each supernatant. The 3 supernatants are then pooled and lyophilized such that multiple 100µg lyophilized aliquots are obtained. AEF is used for both cell culture studies (aim 1) and experimental AA amyloid induction in a mouse model (aim 2) as described below. We have found that 10 amyloidotic spleens yield approximately 4 mg of AEF (enough for injections for 40 animals; i.e. 100µg per animal as described herein.

### Production and Purification of Recombinant SAA2:

Mouse recombinant SAA2 corresponding to the SAA in mice of the CE/J strain (CE/J SAA) is produced in Escherichia coli BL834 cells, using the pET-21a vector as previously described (Kluve-Beckerman et al, 1997). Purification from E. coli lysates are accompanied by Sepharose CL-6B chromatography in 4 M guanidine, 0.05 M Tris-HCl (pH 8.2) followed by chromatofocusing over a range of pH 8 to pH 5 in 6M urea (Kluve-Beckerman et al, 1997). Final preparations are precipitated in ammonium sulfate (80% saturated), extensively dialyzed against water, and lyophilized.

### Induction of AA Amyloid Formation in Cell Culture:

To induce AA amyloid formation and deposition in macrophage/peritoneal cells in culture, recombinant mouse SAA2 (at a final concentration of 140µg/ml), and AEF (at a final concentration of 12 µg/ml) is used. SAA (7µl) is added directly to the medium (350µl) from a 7-10 mg/ml stock solution prepared by dissolving purified, lyophilized recombinant SAA2 in 6M urea, 25 mM HEPES, pH 7.2. Concentrations of stock solutions are adjusted after analysis by SDS-PAGE and densitometric quantitation of Coomassie-blue stained SAA bands. AEF stock solutions (2 mg/ml) is thoroughly mixed to resuspend precipitated material before withdrawal of an aliquot (2µl) for addition to culture medium (350µl).

### Cell Viability Assay:

To confirm cell viability of macrophage cultures following the induction of AA amyloid (as described above), exclusion of trypan blue is used in some cultures (at days 5,10 and 20). Before staining, cells are rinsed 3 times with serum-free RPMI. They are then covered for 1 minute with a solution of 2% (w/v) trypan blue in PBS (Perry et al, 1997). Immediately after the trypan blue is removed, cells are fixed with 4% (w/v) paraformaldehyde, pH 7.5, for 10 minutes at room temperature, rinsed four times with PBS with gentle shaking, and examined microscopically before staining with Congo red or Thioflavin S.

### Macrophage Phagocytotic Activity:

In order to assess and confirm phagocytotic activity of macrophage cells in culture (at days 5,10 and 20 following AA amyloid induction), a latex bead uptake assay is also employed. Blue-dyed polystyrene latex beads, 0.8µm in diameter (Sigma) is suspended in serum-free RPMI. at a concentration of 7.2 X 10⁸/ml. Cells are rinsed 3 times with serum-free RPMI and then incubated for 30 minutes with 300µl of the bead suspension. During the incubation, chamber slides are tightly wrapped in Parafilm and shaken gently in a water bath at 37°C. The bead suspension is removed, and the cells are rinsed a minimum of 10 times with RPMI. Cells are then fixed with formalin and stained with hematoxylin.

### Congo Red Staining:

To confirm and quantitate fibrillar AA amyloid deposition in culture, cells at 5,10 and 20 days (post AA amyloid induction) are fixed in ice-cold 100% methanol and then stained for 45 minutes with Congo red prepared in alkaline 80% ethanol (Puchtler et al, 1962). After several quick dips in water, slides are immersed in hematoxylin for 2 minutes. Slides are then dipped once in acidified 70% ethanol, several times in water, and once in a 1% solution of NaOH. Dehydration is accomplished by washing sequentially in 95% ethanol and 100% ethanol. Slides are cleared in xylene, and coverslips are applied using Permount. The extent of Congo red staining (i.e. red/green birefringence as viewed under polarized light) is quantitatively determined using an imaging system as described herein. Previous studies using this culture system indicated that Congo red-positive material remains attached to chamber slides throughout the culture period (2-24 days)(Kluve-Beckerman et al, 1999). AA amyloid accumulation in culture has been previously demonstrated to occur within 2 days following addition of SAA2 and AEF (Kluve-Beckerman et al, 1999).

### Thioflavin S Fluorescence:

Fibrillar AA amyloid deposits are detected and analyzed flowing staining with Thioflavin S (Elghetany and Saleem, 1988). Thioflavin S fluorescence is quantitated using the image analysis system as described herein.

### Quantitation of %AA Amyloid Burden in Cell Culture by Image Analysis:

In order to quantitate the % amyloid burden present in cell cultures following AA amyloid formation and deposition (at days 5, 10, and 20), we use a quantitative method involving image analysis. Briefly, slides stained with Congo redor Thioflavin S are viewed under polarized light, or fluorescent light, respectively. 5-7 slides per treatment group as described herein are used to determine the % amyloid burden at a given magnification (X100). Quantitative image analysis is performed using an Image Pro Plus image analysis system (Media Cybernetics) linked to a Zeiss Axioskop 20 microscope (with polarization and fluorescence capabilities) through a CCD video camera. The images of fibrillar AA amyloid (polarized or fluorescent) sections are stored in a buffer and a specific region (i.e. at a magnification of X100) is manually outlined and the total pixel area occupied by the amyloid structures determined. A monochromatic-based threshold is used to select pixels corresponding to polarized or immunofluorescent structures. The % of the region occupied by the labeled pixels (i.e. red/green for Congo red; yellow/green for Thioflavin S) are calculated. For all image analyses, the treatment status of cultures are unknown to the observer. Mann-Whitney nonparametric analysis is performed using Statview software (SAS Institute, Cary, NC). Reductions of amyloid loads caused by treatment with polyhydroxylated aromatic compounds (as described below) can be quantitatively determined using the above described methodology. We have successfully utilized this imaging system (using Congo red staining; Thioflavin S fluorescence; or a biotinylated-Aß antibody) to determine % amyloid load, number of amyloid plaques, and plaque size, in various brain regions in transgenic mouse models of Alzheimer's disease. Using this method, we also quantitate the number of macrophages *in vitro* (at a magnification of X100) that contain amyloid-laden structures. Previous studies by Kluve-Beckerman et al (1999) demonstrate that using the cell culture system as described above, many of the macrophages accumulate fibrillar AA amyloid within the cytoplasm of macrophage cells.

### Testing of Polyhydroxylated Aromatic Compounds for Inhibition of AA Amyloid Formation and Deposition:

Our data suggests that certain polyhydroxylated aromatic compounds possess potent amyloid inhibitory activity. Commercially available polyphenolic compounds believed suitable for inhibition of fibrillar AA amyloid formation and deposition are disclosed below.

For structure-activity relationship studies, these readily available polyphenolic compounds with hydroxy groups situated at various positions on aromatic rings may be purchased from commercial sources. Initial studies testing the toxicity of these compounds indicate that they usually demonstrate limited toxicity, even at high dosages. Quantitation of % AA amyloid burden as described herein is used to assess the ability of various polyhydroxylated aromatic compounds to inhibit/disrupt AA amyloid formation and deposition in culture.

Representative compounds disclosed and believed to be effective anti-amyloid and anti alpha-synuclein / NAC inhibitors / disrupters include EDTA (C₁₀H₁₆N₂O₈ MW 292.2 used as a negative control only), and commercially available polyphenolic compounds with a variety of phenolic substitution patterns including: Epicatechin (C₁₅H₁₄O₆; MW 290.3), Catechin hydrate (C₁₅H₁₄O₆; MW 290.3), Epigallocatechin (C₁₆H₁₄0₇; MW 306.3, Epigallocatechin Gallate (C₂₂H₁₈O₁₁; MW 458.4), Epicatechin Gallate (C₂₂H₁₈O₁₀; MW 442.4), Gallocatechin Gallate (C₂₂H₁₈O₁₁; MW 458.4), Chlorogenic acid (C₁₆H₁₈O₉; MW 354.3), Hematoxylin (C₁₆H₁₄O₆; MW 302.3), Phloroglucide (C₁₂H₁₀O₆; MW 234.2), Propyl gallate (C₁₀H₁₂O₆; MW 212.2), Gallic acid ethyl ester (C₉H₁₀O₅; MW 198.2), Gallic acid (C₇HₑO₆; MW 170.1), 3,4,5,-trihydroxy-benzamide hydrate (C₇H₇NO₃; MW 169.1), 5-hydroxydopamine (C₈H₁₁NO₃; MW 205.6), 1,2,4-benzenetriol (C₆H₆O₈; MW 126.1), Ellagic acid (C₁₄HₐO₈; MW 302.2), Quercetin (C₁₀H₁₂O₅; MW 338.3), Pyrocatechol (C₆H₆O₂; MW 110.1), Tannic acid (C₇₆H₅₂O₄₆; MW 1701.2), Pyrogallol (C_{B}H₆O₃; MW 126.1), and p-aminosalicylic acid (C₇H₇NO₃; MW 153.1).

All of these compounds are commercially available from Sigma (St. Louis, MO), Fluka (St. Louis, MO) or from Acros Organic (subsidiary of Fisher Scientific, Palatine, IL). As an example of SAR type studies we compare compounds that contain three adjacent hydroxyl groups on an aromatic ring (examples include pyrogallol, 5-hydroxydopamine, gallic acid and propyl gallate) to compounds that contain two adjacent hydroxyl groups (examples include pyrocatechol, quercetin and 1,2,4-benzentriol). Compounds that contain hydroxyl groups that are spaced at least 2 carbons apart (such as phloroglucide) are also evaluated in cell culture. These studies help determine phenolic structural features that contribute to the inhibition of AA fibrillogenesis formation and/or deposition. Synthesis of chemical analogs to the most potent inhibitors identified herein are also contemplated. These studies provide good insights into the structural elements required for the observed bioactivity.

For cell culture studies, an initial high dosage of test compounds (to be determined empirically) is used to determine if the compounds can inhibit AA amyloid formation and/or deposition, as determined by methods as described below and elsewhere herein. For these studies, each polyhydroxylated aromatic compound is added to cultures at the start of AA amyloid induction (i.e. AEF + SAA2 administration). Cell culture media containing each individual test compound is replaced every 2-3 days. % amyloid burden are quantitated at days 5 and 10 following AA amyloid induction and test compound treatment. Comparisons are directly made to the % amyloid burden in cultures only induced for AA amyloid deposition (i.e. no test compound treatment). Those identified to show promise as inhibitors of fibrillar AA amyloid formation and/or deposition at a single high dose, are tested more extensively, and at increasing concentrations (i.e. low dose, medium dose and high dose; to be determined empirically) to confirm a dose-dependent inhibition of fibrillar AA amyloid formation and/or deposition. Selected compounds identified by screening using the cell culture methods as described above, are then tested in an experimental mouse model of AA amyloidosis.

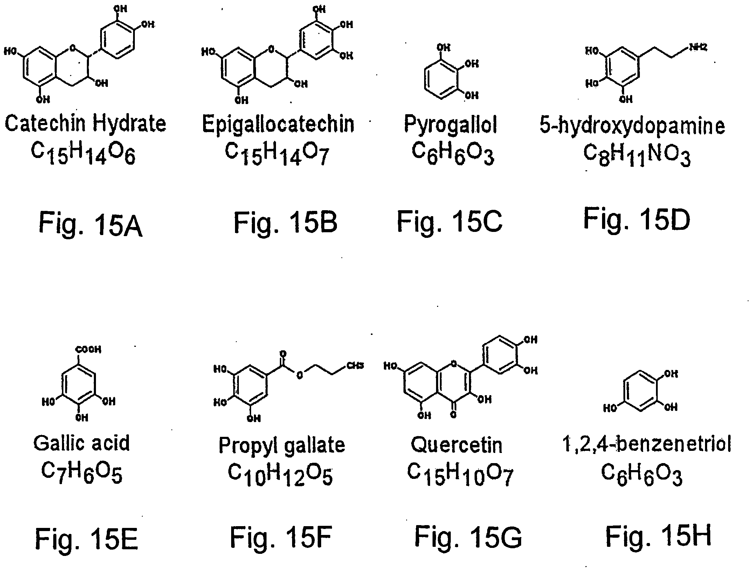

Figure 15 shows structure-activity relationship (SAR) studies of different commercially available polyhydroxylated aromatic compounds. Shown are 8 examples of the structures of different commercially available polyphenolic compounds which differ by the placement of the hydroxyl groups on the aromatic ring to be used for SAR screening studies.

### Quantitation of AA Amyloid Using SDS-PAGE and Immunoblotting:

In addition to the quantitation of % amyloid burden in culture following Congo red staining and Thioflavin S fluorescence using image analysis, AA amyloid accumulation in cell layer and media is determined using SDS-PAGE followed by immunoblotting as previously described (Kluve-Beckerman et al, 1999). Briefly, cell layers are rinsed three times with serum-free RPMI, scraped into PBS, pelleted by centrifugation, and solubilized in SDS sample buffer. For analysis of AA amyloid in culture media derived from cells, 15µl aliquots of media is added directly to SDS sample buffer. Triplicate samples are subjected to tricine-SDS-PAGE as previously described (Schagger and von Jago, 1987). Using this method, separating spacing, and stacking layers contain 16.5%,10% and 4% polyacrylamide, respectively. A monoclonal antibody against AA amyloid protein (Serotek), and the technique of enhanced chemoluminescence (ECL) Western blotting is employed according to the manufacturer's recommendations (Amersham Life Science). Using the methods as described above, SAA2 usually appears as multiple bands art ∼12-14 kDa and higher, whereas AA protein appears as a major band at ∼8.5 kDa (Kluve-Beckerman et al, 1999). Initially once the cell cultures are established, N-terminal sequencing of the major band at ∼8.5 kDa is implemented to confirm the presence of AA protein. We have previously used the method of electrophoresis, electroelution and amino acid sequencing to identify other peptide sequences for other in-house projects.

Once this system is established, we use scanning densitometry to determine the extent of AA amyloid protein in cell layer and media following induction by SAA2 + AEF (at 5 and 10 days post AA amyloid induction), in the absence or presence of a high dose of each polyhydroxylated aromatic compound (as specified above). The average of 3 scans per sample (with samples performed on SDS-PAGE in triplicate) is used to determine the levels of AA amyloid protein in cell layer and media under the various conditions. Statistical analysis comparing scanning densitometric analysis in the absence or presence of polyphenolic compounds is based on paired Student's t-tests with data shown as mean +/- S.E. Significance is reported at the 95% (p<0.05), 99% (p<0.01) and 99.9% (p<0.001) confidence levels. ANOVA is also used as necessary.

### Example 13

### Identification of New AA Amyloid Inhibitors - Animal Studies

Studies suggest that certain polyhydroxylated aromatic compounds, such as epicatechin, appear to serve as potent inhibitor of fibrillar AA amyloid formation and deposition *in vivo.* We display animal model studies to determine the efficacy of selected polyhydroxylated aromatic compounds in an experimental mouse model ofAA amyloidosis. Groups of CBA/J mice (n= 8 per group) is first pre-treated with different polyhydroxylated aromatic compounds for a 7-day pre-treatment period at a high non-toxic dose (to be determined empirically). Animals are induced for fibrillar AA amyloidosis by a single subcutaneous injection of AgNO₃, and a single tail vein injection of 100µg of AEF. Treatment during the amyloid induction phase with polyhydroxylated aromatic compounds continues for another 14 days before sacrifice (treatment time = 21 days total). Spleen, livers and kidneys are obtained from each animal, and the % amyloid burden in each organ is quantitated using image analysis. In addition, immunohistochemistry is implemented using antibodies against AA amyloid protein, SAA, heparan sulfate proteoglycans/ glycosaminoglycans, amyloid P component, and ApoE to assess the effects of test compounds on the accumulation AA amyloid/SAA, and other known AA amyloid co-factors.

### Methodology:

### Treatment Groups:

Groups (n= 8 per group) of 8-week old CBA/J female mice (Baxter Labs) is used for the animal model studies. Initially each animal is pre-treated daily by oral gavage (in a 0.5 ml volume) with a specific polyhydroxylated aromatic compound (selected from results of testing above) at an initial high non-toxic dose (to be determined empirically) for a 7-day pretreatment period. Typically our previous low to high dosages with polyhydroxylated aromatic compounds given orally, range from a low dose of 25 mg/kg/day, to a high dose of 500 mg/kg/day. Equivalent dosages are used to compare different polyhydroxylated aromatic compounds taking into account compound toxicity and compound solubility (in PBS). Oral gavage is accomplished using a 22-gauge oral dosing needle (Popper) of test compounds dissolved in a 0.50 ml of PBS (pH 7.4). On day 8, all animals are induced for AA amyloidosis by a single subcutaneous injection of 0.5ml of 3% AgNO₃ (Fisher) in double-distilled deionized water. This is followed by a single tail-vein injection of 100µg of AEF in double-distilled deionized distilled water. Animals are treated for an additional 14 days with each of the specific polyhydroxylated compounds. One group of 12 mice is induced for AA amyloidosis (by AgNO₃ + AEF as described above) and is treated orally with PBS (pH 7.4) only, in the absence of any test compounds. At the end of the 21-day treatment period, animals are sacrificed by overdose with 0.1ml of euthosol, and the spleen, liver and kidneys from each animal is harvested for staining and immunohistochemistry. The number of animals per group (n=8 for test compounds; n=12 for control saline group) are determined from data obtained, and power calculations based on an alpha level of 0.05 (two-sided) and standard deviations of 0.5, which gave a power of 94%.

### AEF Production:

AEF is prepared as described above.

### Fixation and Tissue Preparation:

Fixation and tissue preparation is as previously described (Snow et al, 1991). Briefly, the spleen, liver and kidneys are removed from each animal, fixed in 90% ethanol, and 10% formaldehyde for 24 hours at -20°C, embedded in paraffin and sectioned at 25µM (thick sections needed for amyloid load quantitation as described below). Approximately 50-75 sections are cut per animal.

### Congo Red Staining and Thioflavin S Fluorescence:

Fibrillar AA amyloid accumulation is detected by Congo red staining (Puchtler et al, 1962) and Thioflavin S fluorescence (Elghetany and Saleem, 1988) as described above.

### Quantitation of Amyloid Burden in Tissues:

Image analysis is also used to quantitate the % amyloid burden present in spleen, liver and kidney, of each animal. The % amyloid load in each organ is quantified as assessed following staining with Congo red (as viewed under polarized light) or Thioflavin S (as viewed under fluorescent light). 5-7 equally spaced slides per animal per stain (in each treatment group) are used to determine the % amyloid load at a given magnification (X100). Quantitative image analysis of amyloid load in a given organ (and at a specific magnification) is performed using the image analysis system as described above. Potential reductions of amyloid load is quantitatively determined for each polyhydroxylated aromatic compound for each organ/animal. Certain test compounds are expected to reduce amyloid load by 40-60%.

### Immunohistochemistry:

Tissue sections are also immunostained to detect important AA amyloid protein markers, and known AA amyloid-associated co-factors. A monoclonal antibody against the AA amyloid protein (Serotek) is used to confirm the location of AA amyloid deposits, whereas a polyclonal against SAA (Serotek) is used to detect the precursor protein, SAA. The presence of perlecan (a specific heparan sulfate proteoglycan implicated in AA amyloidosis)(Snow et al, 1991) is detected using a monoclonal antibody against the perlecan core protein (known as HK-102; generous gift of Dr. Koji Kimata, Japan)(Snow et al, 1994), whereas a phage display generated periplasmic fraction antibody (known as HS4C3; generous gift of Dr. Van Kuppevelt)(van Kuppevelt et al, 1998) against a specific epitope of heparan sulfate GAG chains are used to determine the co-localization of heparan sulfate GAGs. Amyloid P component is detected using a polyclonal antibody (Research Diagnostics), whereas ApoE is detected using a polyclonal antibody (Research Diagnostics).

For antibody localization we use standard fluorescence techniques (Basgen et al, 1989; Mosedale et al, 1996). The primary antibody for immunohistochemical staining is used through a series of dilutions to obtain the best specificity with the least background staining. Controls for positive immunostaining consist of sections treated with either 1) preabsorption of the primary antibody with excess antigen (if available), 2) a different primary antibody of the same Ig class and species, and/or 3) PBS + 1% bovine serum albumin instead of the primary antibody (to ensure that there is no non-specific binding of the secondary antibodies employed).

### Pharmacology and Utility

The disclosed compounds act to inhibit or prevent amyloid fibril formation, inhibit or prevent amyloid fibril growth, and/or cause disassembly, disruption, and/or disaggregation of preformed amyloid fibrils and amyloid protein deposits. Their activity can be measured *in vitro* by methods such as those discussed in Examples 1 through 4, while their activity in vivo against amyloidoses can be measured in animal models, such as those of Alzheimer's disease, Parkinson's disease, systemic AA amyloidosis, type 2 diabetes and others.

The compounds also act to inhibit or prevent alpha-synuclein/NAC fibril formation, inhibit or prevent alpha-synuclein/NAC fibril growth, and/or cause disassembly, disruption, and/or disaggregation of preformed alpha-synuclein/NAC fibrils and alpha-synuclein/NAC-associated protein deposits. Their activity can be measured *in vitro* by methods such as those discussed in Example 4 above.

The therapeutic ratio of a compound can be determined, for example, by comparing the dose that gives effective anti-fibril (anti-amyloid or anti-alpha-synuclein/NAC activity in a suitable in vivo model in a suitable animal species such as the mouse, with the dose that gives significant weight loss (or other observable side-effects) in the test animal species.

### Pharmaceutical compositions and administration

In general, compounds are administered in pure isolated form in therapeutically effective amounts by any of the usual modes known in the art, either singly or in combination with at least one other compound and/or at least one other conventional therapeutic agent for the disease being treated. A therapeutically effective amount may vary widely depending on the disease, its severity, the age and relative health of the animal being treated, the potency of the compound(s), and other factors. As anti-fibril agents, therapeutically effective amounts of compounds may range from 10-1000mg/Kg body weight; for example, 10-100 mg/Kg body weight. A person of ordinary skill in the art will be able without undue experimentation, having regard to that skill and this disclosure, to determine a therapeutically effective amount of a compound of this invention for the treatment of amyloidosis.

In general, compounds are administered as pharmaceutical compositions by one of the following routes: oral, topical, systemic (e.g. transdermal, intranasal, or by suppository), or parenteral (e.g. intramuscular, subcutaneous, or intravenous injection). Compositions may take the form of tablets, pills, capsules, semisolids, powders, sustained release formulations, solutions, suspensions, elixirs, aerosols, or any other appropriate compositions; and comprise at least one compound in combination with at least one pharmaceutically acceptable excipient. Suitable excipients are well known to persons of ordinary skill in the art, and they, and the methods of formulating the compositions, may be found in such standard references as Alfonso AR: Remington's Pharmaceutical Sciences, 17th ed., Mack Publishing Company, Easton PA, 1985. Suitable liquid carriers, especially for injectable solutions, include waster, aqueous saline solution, aqueous dextrose solution, and glycols.

In particular, the compound(s) can be administered, orally, for example, as tablets, troches, lozenges, aqueous or oily suspension, dispersible powders or granules, emulsions, hard or soft capsules, or syrups or elixirs. Compositions intended for oral use may be prepared according to any method known in the art for the manufacture of pharmaceutical compositions and such compositions may contain one or more agents selected from the group consisting of sweetening agents, flavoring agents, coloring agents and preserving agents in order to provide pharmaceutically elegant and palatable preparations.

Tablets contain the compound in admixture with non-toxic pharmaceutically acceptable excipients which are suitable for the manufacture of tablets. These excipients may be for example, inert diluents, such as calcium carbonate, sodium carbonate, lactose, calcium phosphate or sodium phosphate; granulating and disintegrating agents, for example, maize starch or alginic acid; binding agents, for example, maize starch, gelatin or acacia, and lubricating agents, for example, magnesium stearate or stearic acid or tale. The tablets may be uncoated or they may be coated by known techniques to delay disintegration and absorption in the gastrointestinal tract and thereby provide a sustained action over a longer period. For example, a time delay material such as glycerol monostearate or glycerol distearate may be employed. Formulations for oral use may also be presented as hard gelatin capsules wherein the compound is mixed with an inert solid diluent, for example, calcium carbonate, calcium phosphate or kaolin, or as soft gelatin capsules wherein the active ingredient is mixed with water or an oil medium, for example, peanut oil, liquid paraffin or olive oil.

Aqueous suspensions contain the compound in admixture with excipients suitable for the manufacture of aqueous suspensions. Such excipients are suspending agents, for example, sodium carboxymethylcellulose, methylcellulose, hydroxypropylmethyl cellulose, sodium alginate, polyvinylpyrrolidone, gum tragacanth and gum acacia; dispersing or wetting agents may be naturally occurring phosphatides, for example lecithin, or condensation products of an alkylene oxide with fatty acids, for example polyoxyethylene stearate, or condensation products of ethylene oxide with long chain aliphatic alcohols, for example, heptadecaethyleneoxycetanol, or condensation products of ethylene oxide with partial esters derived from fatty acids such as hexitol such as polyoxyethylene sorbitol monooleate, or condensation products of ethylene oxide with partial esters from fatty acids and a hexitol annhydrides, for example, polyethylene sorbitan monooleate. The aqueous suspensions may also contain one or more preservatives, for example, ethyl or n-propyl p-hydroxybenzoate, one or more coloring agents, one or more flavoring agents, or one or more sweetening agents, such as sucrose or saccharin.

Oily suspensions may be formulated by suspending the compound in a vegetable oil, for example arachis oil, olive oil, sesame oil, or coconut oil or in a mineral oil such as liquid paraffin. The oily suspensions may contain a thickening agent, for example beeswax, hard paraffin or cetyl alcohol. Sweetening agents, such as those set forth below, and flavoring agents may be added to provide a palatable oral preparation. These compositions may be preserved by the addition of an antioxidant such as ascorbic acid. Dispersible powders and granules suitable for preparation of an aqueous suspension by the addition of water provide the active ingredient in admixture with a dispersing or wetting agent, a suspending agent and one or more preservatives. Suitable dispersing or wetting agents and suspending agents are exemplified by those already described above. Additional excipients, for example sweetening, flavoring and agents, may also be present.

The compounds may also be in the form of oil-in-water emulsions. The oily phase may be a vegetable oil, for example olive oil or arachis oils, or a mineral oil, for example liquid paraffin or mixtures of these. Suitable emulsifying agents may be naturally-occurring gums, for example gum acacia or gum tragacanth, naturally occurring phosphatides, for example soy bean, lecithin, and occurring phosphatides, for example soy bean, lecithin, and esters or partial esters derived from fatty acids and hexitol anhydrides, for example sorbitan monooleate, and condensation products of the said partial esters with ethylene oxide, for example polyoxyethylene sorbitan monooleate. The emulsion may also contain sweetening and flavoring agents. Syrups and elixirs may be formulated with sweetening agents, for example, glycerol, sorbitol or sucrose. Such formulations may also contain a demulcent, a preservative and flavoring and coloring agents.

The compounds can also be administered by injection or infusion, either subcutaneously or intravenously, or intramuscularly, or intrasternally, or intranasally, or by infusion techniques in the form of sterile injectable or oleaginous suspension. The compound may be in the form of a sterile injectable aqueous or oleaginous suspensions. These suspensions may be formulated according to the known art using suitable dispersing of wetting agents and suspending agents which have been described above. The sterile injectable preparation may also be a sterile injectable solution or suspension in a non-toxic parenterally-acceptable diluent or solvent for example, as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium. For this purpose any bland fixed oils may be conventionally employed including synthetic mono- or diglycerides. In addition fatty acids such as oleic acid find use in the preparation of injectables.

Dosage regimens can be adjusted to provide the optimum therapeutic response. For example, several divided dosages may be administered daily or the dosage may be proportionally reduced as indicated by the exigencies of the therapeutic situation.

It is especially advantageous to formulate the compounds in dosage unit form for ease of administration and uniformity of dosage. Dosage unit form as used herein refers to physically discrete units suited as unitary dosages for the subjects to be treated; each containing a therapeutically effective quantity of the compound and at least one pharmaceutical excipient. A drug product will comprise a dosage unit form within a container that is labeled or accompanied by a label indicating the intended method of treatment, such as the treatment of an amyloid disease, such as Alzheimer's disease, or of a disease associated with alpha-synuclein fibril formation, such as Parkinson's disease. A "therapeutically effective dosage" preferably inhibits amyloidosis or a disease associated with alpha-synuclein fibril formation in a patient by at least 20, more preferably by at least 40%, even more preferably by at least 60%, and still more preferably by at least 80%, relative to untreated subjects.

### Preparation of the Compounds

Many of the compounds used in disclosed methods are well known to the art and are commercially available. They may be briefly described in such references as the Merck Index, 12th edition, Merck & Co., Inc., Whitehouse Station, New Jersey, 1996 (which typically provides a reference to a synthesis or isolation), and may be found in chemical catalogs, such as those of commercial suppliers as described herein.

### INDUSTRIAL APPLICABILITY

The invention finds worldwide utility in that it provides therapeutic relief and diagnostic assistance in treating Parkinson's disease and Lewy body disease, by use of a catechin.

In compliance with the statute, the invention has been described in language more or less specific as to structural features. It is to be understood, however, that the invention is not limited to the specific features shown, since the means and construction shown comprise preferred forms of putting the invention into effect. The invention is, therefore, claimed in any of its forms or modifications within the legitimate and valid scope of the appended claims, appropriately interpreted in accordance with the doctrine of equivalents.

## Claims

1. The use of a catechin in the manufacture of a medicament for treating Parkinson's disease or Lewy body disease, wherein the catechin is selected from the group consisting of catechin, epicatechin, gallocatechin gallate, epigallocatechin gallate, epigallocatechin, and epicatechin gallate, and pharmaceutically acceptable salts of the foregoing catechins.

2. A catechin selected from the group consisting of catechin, epicatechin, gallocatechin gallate, epigallocatechin gallate, epigallocatechin, and epicatechin gallate, and pharmaceutically acceptable salts of the foregoing catechins, for treating Parkinson's disease or Lewy body disease.

3. The use of claim 1, wherein the medicament is for oral administration, parenteral injection, intravenous injection, subcutaneous injection, intramuscular injection, topical administration, or aerosol spray administration.

4. The catechin of claim 2, which is for oral administration, parenteral injection, intravenous injection, subcutaneous injection, intramuscular injection, topical administration, or aerosol spray administration.

5. A method of treatment of alpha-synuclein or NAC fibrillogenesis in an *in vitro* environment, the method comprising the step of administering into the *in vitro* environment a therapeutically effective amount of a catechin selected from the group consisting of catechin, epicatechin, gallocatechin gallate, epigallocatechin gallate, epigallocatechin, and epicatechin gallate, and pharmaceutically acceptable salts of the foregoing catechins.

6. A pharmaceutical composition for use in treating Parkinson's disease or Lewy body disease, comprising a therapeutically effective amount of a catechin selected from the group consisting of catechin, epicatechin, gallocatechin gallate, epigallocatechin gallate, epigallocatechin, and epicatechin gallate, and pharmaceutically acceptable salts of the foregoing catechins and a pharmaceutically acceptable carrier, diluent, or excipient, wherein the therapeutically effective amount of the catechin comprises a dosage in the range of about 0.1 to 500 mg/kg of body weight of the subject.

7. The composition of claim 6, wherein the therapeutically effective amount of the catechin comprises a dosage in the range of about 1.0 to 100 mg/kg of body weight of the subject.

8. The composition of claim 6 or claim 7, comprising a mixture of two or more of the catechins selected from the group consisting of catechin, epicatechin, gallocatechin gallate, epigallocatechin gallate, epigallocatechin, and epicatechin gallate, and pharmaceutically acceptable salts of the foregoing catechins.

9. The composition of claim 6, 7 or 8, wherein the catechin selected is in substantially pure isolated form.

## Patentansprüche

1. Verwendung eines Catechins in der Herstellung eines Medikaments zum Behandeln von Parkinson-Krankheit oder Lewy-Körperchen-Krankheit, wobei das Catechin ausgewählt ist aus der Gruppe bestehend aus Catechin, Epicatechin, Gallocatechingallat, Epigallocatechingallat, Epigallocatechin und Epicatechingallat, und pharmazeutisch annehmbarer Salze der vorangehenden Catechine.

2. Catechin, ausgewählt aus der Gruppe bestehend aus Catechin, Epicatechin, Gallocatechingallat, Epigallocatechingallat, Epigallocatechin und Epicatechingallat, und pharmazeutisch annehmbare Salze der vorangehenden Catechine zum Behandeln von Parkinson-Krankheit oder Lewy-Körperchen-Krankheit.

3. Verwendung nach Anspruch 1, wobei das Medikament für orale Verabreichung, parenterale Injektion, intravenöse Injektion, subkutane Injektion, intramuskuläre Injektion, topische Verabreichung oder Aerosol-Spray-Verabreichung ist.

4. Catechin nach Anspruch 2, welches für orale Verabreichung, parenterale Injektion, intravenöse Injektion, subkutane Injektion, intramuskuläre Injektion, topische Verabreichung oder Aerosol-Spray-Verabreichung ist.

5. Verfahren der Behandlung von Alpha-Synuclein oder NAC-Fibrillogenese in einer *in vitro* Umgebung, wobei das Verfahren den Schritt des Verabreichens einer therapeutisch wirksamen Menge eines Catechins, ausgewählt aus der Gruppe bestehend aus Catechin, Epicatechin, Gallocatechingallat, Epigallocatechingallat, Epigallocatechin und Epicatechingallat und pharmazeutisch annehmbarer Salze der vorangehenden Catechine in die *in vitro* Umgebung umfasst.

6. Pharmazeutische Zusammensetzung zur Verwendung beim Behandeln von Parkinson-Krankheit oder Lewy-Körperchen-Krankheit, welche eine therapeutisch wirksame Menge eines Catechins, ausgewählt aus der Gruppe bestehend aus Catechin, Epicatechin, Gallocatechingallat, Epigallocatechingallat, Epigallocatechin und Epicatechingallat und pharmazeutisch annehmbarer Salze der vorangehenden Catechine und einen pharmazeutisch annehmbaren Träger, Verdünnungsmittel oder Arzneimittelträger umfasst, wobei die therapeutisch wirksame Menge des Catechins eine Dosierung im Bereich von etwa 0,1 bis 500 mg/kg Körpergewicht des Subjekts umfasst.

7. Zusammensetzung nach Anspruch 6, wobei die therapeutisch wirksame Menge des Catechins eine Dosierung im Bereich von etwa 1,0 bis 100 mg/kg Körpergewicht des Subjekts umfasst.

8. Zusammensetzung nach Anspruch 6 oder Anspruch 7, welche ein Gemisch aus zwei oder mehreren der Catechine, ausgewählt aus der Gruppe bestehend aus Catechin, Epicatechin, Gallocatechingallat, Epigallocatechingallat, Epigallocatechin und Epicatechingallat und pharmazeutisch annehmbaren Salzen der vorangehenden Catechine umfasst.

9. Zusammensetzung nach Anspruch 6, 7 oder 8, worin des gewählte Catechin seine im Wesentlichen reine isolierte Form hat.

## Revendications

1. Utilisation d'une catéchine dans la fabrication d'un médicament pour traiter la maladie de Parkinson ou la maladie à corps de Lewy, dans laquelle la catéchine est choisie dans le groupe constitué de catéchine, d'épicatéchine, de gallate de gallocatéchine, de gallate d'épigallocatéchine, d'épigallocatéchine, et de gallate d'épicatéchine, et des sels pharmaceutiquement acceptables desdites catéchines.

2. Catéchine choisie dans le groupe constitué de catéchine, d'épicatéchine, de gallate de gallocatéchine, de gallate d'épigallocatéchine, d'épigallocatéchine, et de gallate d'épicatéchine, et des sels pharmaceutiquement acceptables desdites catéchines, pour traiter la maladie de Parkinson ou la maladie à corps de Lewy.

3. Utilisation selon la revendication 1, dans laquelle le médicament est destiné à une administration par voie orale, une injection parentérale, une injection intraveineuse, une injection sous-cutanée, une injection intramusculaire, une administration topique, ou une administration par spray aérosol.

4. Catéchine selon la revendication 2, destinée à une administration par voie orale, une injection parentérale, une injection intraveineuse, une injection sous-cutanée, une injection intramusculaire, une administration topique, ou une administration par spray aérosol.

5. Procédé de traitement d'alpha-synucléine ou de fibrillogénèse NAC dans un environnement in vitro, le procédé comprenant l'étape consistant à administrer dans l'environnement in vitro une quantité thérapeutiquement efficace d'une catéchine choisie dans le groupe constitué de catéchine, d'épicatéchine, de gallate de gallocatéchine, de gallate d'épigallocatéchine, d'épigallocatéchine, et de gallate d'épicatéchine, et des sels pharmaceutiquement acceptables desdites catéchines.

6. Composition pharmaceutique à utiliser dans le traitement de la maladie de Parkinson ou de la maladie à corps de Lewy, comprenant une quantité thérapeutiquement efficace d'une catéchine choisie dans le groupe constitué de catéchine, d'épicatéchine, de gallate de gallocatéchine, de gallate d'épigallocatéchine, d'épigallocatéchine, et de gallate d'épicatéchine, et des sels pharmaceutiquement acceptables desdites catéchines et un support, un diluant, ou un excipient pharmaceutiquement acceptable, dans lequel la quantité thérapeutiquement efficace de la catéchine comprend un dosage dans la gamme d'environ 0,1 à 500 mg/kg de masse corporelle du sujet.

7. Composition selon la revendication 6, dans laquelle la quantité thérapeutiquement efficace de la catéchine comprend un dosage dans la gamme d'environ 1,0 à 100 mg/kg de masse corporelle du sujet.

8. Composition selon la revendication 6 ou la revendication 7, comprenant un mélange de deux ou plus des catéchines choisies dans le groupe constitué de catéchine, d'épicatéchine, de gallate de gallocatéchine, de gallate d'épigallocatéchine, d'épigallocatéchine, et de gallate d'épicatéchine, et des sels pharmaceutiquement acceptables desdites catéchines.

9. Composition selon la revendication 6, 7 ou 8, dans laquelle la catéchine choisie est sous une forme isolée sensiblement pure.
